(19) Europäisches Patentamt
European Patent Office
Office européen des brevets

(11) **EP 4 223 878 A1**

(12) **EUROPEAN PATENT APPLICATION**
published in accordance with Art. 153(4) EPC

(43) Date of publication:
**09.08.2023  Bulletin 2023/32**

(21) Application number: **21875877.9**

(22) Date of filing: **01.10.2021**

(51) International Patent Classification (IPC):
*C12N 15/13* (2006.01)          *A61K 39/215* (2006.01)
*A61P 31/14* (2006.01)          *C07K 16/10* (2006.01)
*C07K 16/46* (2006.01)

(52) Cooperative Patent Classification (CPC):
**A61K 38/02; A61K 39/215; A61K 39/395;**
**A61P 31/14; C07K 16/00; C07K 16/10;**
**C07K 16/46; C12N 15/62; G01N 33/531;**
**G01N 33/569**

(86) International application number:
**PCT/JP2021/036446**

(87) International publication number:
**WO 2022/071581 (07.04.2022 Gazette 2022/14)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: 02.10.2020  JP 2020168078
          05.11.2020  JP 2020185268
          03.12.2020  JP 2020201212
          20.05.2021  JP 2021085698
          19.08.2021  JP 2021134372
          15.09.2021  JP 2021150604

(71) Applicants:
• **Epsilon Molecular Engineering Inc.**
  **Saitama City, Saitama 338-8570 (JP)**
• **The Kitasato Institute**
  **Tokyo 108-8641 (JP)**
• **Kao Corporation**
  **Chuo-ku**
  **Tokyo 103-8210 (JP)**

(72) Inventors:
• **MASAKI, Hidekazu**
  **Saitama City, Saitama 338-8570 (JP)**
• **KUMACHI, Shigefumi**
  **Saitama City, Saitama 338-8570 (JP)**

• **YONEHARA, Ryo**
  **Saitama City, Saitama 338-8570 (JP)**
• **MATSUMURA, Yuta**
  **Haga-gun, Tochigi 321-3497 (JP)**
• **KAWANO, Hibiki**
  **Tokyo 131-8501 (JP)**
• **TOJO, Takuto**
  **Wakayama-shi, Wakayama 640-8580 (JP)**
• **MORIMOTO, Takuya**
  **Haga-gun, Tochigi 321-3497 (JP)**
• **KATAYAMA, Kazuhiko**
  **Tokyo 108-8641 (JP)**
• **HAGA, Kei**
  **Tokyo 108-8641 (JP)**
• **TODAKA, Reiko**
  **Tokyo 108-8641 (JP)**
• **SAWADA, Akihito**
  **Tokyo 108-8641 (JP)**
• **TAKANO, Tomomi**
  **Towada-shi, Aomori 034-8628 (JP)**

(74) Representative: **Vossius & Partner**
  **Patentanwälte Rechtsanwälte mbB**
  **Siebertstraße 3**
  **81675 München (DE)**

Remarks:
The complete document including Reference Table(s) and the Sequence Listing(s) can be downloaded from the EPO website

(54)  **SARS-COV-2-BINDING PEPTIDE**

(57)    A peptide which binds to SARS-CoV-2 and its usage are provided. The peptide which binds to SARS-CoV-2 comprises one or more structural domain comprising CDR3 consisting of an amino acid sequence of any of SEQ ID NOs: 1 to 9 or an amino acid sequence obtained by substituting at least one amino acid in the amino acid sequence with another amino acid.

EP 4 223 878 A1

**Description**

Field of the Invention

The present invention relates to a peptide that binds to SARS-CoV-2.

Background of the Invention

SARS coronavirus-2 (severe acute respiratory syndrome coronavirus 2: SARS-CoV-2) belongs to the genus beta-coronavirus, which is the same as SARS coronavirus (SARS-CoV) and MERS coronavirus (MERS-CoV), and is a SARS-related coronavirus which is a cause of acute respiratory disease. The virus causing acute respiratory disease that was first confirmed near Wuhan, Hubei Province, China in 2019 was later identified as a novel coronavirus and named as COVID-19. The virus has been causing global epidemic (pandemic) while mutating in various ways.

SARS-CoV-2 includes 4 structural proteins, 16 nonstructural proteins (nsp 1-16), and a large positive-sense RNA genome encoding some accessory proteins, like common coronaviruses. The structural proteins are a spike protein, a nucleocapsid, a membrane protein, and an envelope protein, and the spike protein is constituted of an S1 subunit at the N-terminal responsible for receptor binding and an S2 subunit at the C-terminal responsible for membrane fusion. The S1 subunit is further divided into an N-terminal domain (NTD), a receptor binding domain (RBD), a subdomain 1 (SD1), and a subdomain 2 (SD2). It is known that the S2 subunit is further divided into a fusion peptide (FP), a heptad repeat 1 (HR1), and a heptad repeat 2 (HR2) (see Non Patent Literature 1).

As in the case of SARS-CoV, SARS-CoV-2 binds to a host cell receptor, angiotensin converting enzyme 2 (ACE2), mediated by the spike protein and enters into the cell. The binding to the receptor triggers a conformational change in the spike protein to convert the protein into an activated state. The activated spike protein is cleaved at S1/S2 site by a protease (TMPRSS2 in SARS-CoV and SARS-CoV-2) to release the S1 subunit and expose the FP on the S2 subunit. It is known that the FP is inserted into the target cell membrane and forms the post-fusion conformation by refolding of HR1 and HR2 to promote the viral membrane fusion with the target cell (see Non Patent Literature 2).

Recently, structural analysis of the prefusion conformation of the SARS-CoV-2 trimeric spike, SARS-CoV-2 spike RBD, and full-length human ACE2 by cryo-electron microscopy or crystal analysis has been progressed, and elucidation of the binding mechanism to ACE2, knowledge about an important mutation site on the RBD forming more strong contact with human ACE2, and also insight for the epitope toward identification of a cross-reactive antibody targeting SARS-CoV and the RBD of SARS-CoV2 have been provided. Furthermore, it has been reported based on sugar chain analysis of the SARS-CoV-2 spike protein that the SARS-CoV-2 spike protein includes a large number of glycosylation sites like SARS-CoV and a unique glycosylation site that is not present in SARS-CoV is included, and also reported that such a specific sugar chain has a risk of promoting immune evasion through masking of the epitope (see Non Patent Literature 3).

The spike protein of SARS-CoV-2 plays a role essential for enter of a virus into a host cell and is a main target of a neutralizing antibody. It has been reported that due to functionality and high immunogenicity of the coronavirus S1 subunit, most neutralizing antibodies that have been characterized against coronaviruses target S1, in particular, S1-RBD. Some neutralizing antibodies were developed also during previous epidemics of SARS and MERS infections and were proved to have a possibility of use in therapy of coronavirus infectious disease.

However, it has been reported that many known neutralizing antibodies (such as S230, m396, and 80R) specific to the SARS-CoV RBD do not bind to SARS-CoV-2 even at concentrations up to 1 μM and also that every monoclonal antibody isolated from SARS-CoV-2-infected individuals by sorting single B-cells does not show cross- reaction with the SARS-CoV RBD. Thus, it has been reported that many existing neutralizing antibodies have poor cross-reactivity and that there are present VHH-72-Fc and so on as a single-domain antibody, or an IgG antibody such as 47D11 and S309, showing neutralizing activity to both SARS-CoV and SARS-CoV2 (see Non Patent Literature 3).

Virological diagnosis of COVID-19 is performed by genetic detection (PCR test) of SARS-CoV-2 mainly by polymerase chain reaction (PCR) (hereinafter, referred to as "conventional technology 1").

In contrast, it is also known that viruses can be detected also by testing methods different from PCR. One of them is an antigen test that detects a fragment of protein on the virus surface rather than detects genetic material of the virus (hereinafter, referred to as "conventional technology 2"). Infection can be diagnosed in minutes without an expensive machine, training to master the skills required for inspectors, extensive effort required for inspection, and so on as long as an antigen can be detected.

As other testing methods, for example, serodiagnosis utilizing immunochromatography for detecting a virus-specific antibody in serum and enzyme antibody technique (ELISA) are known (hereinafter, referred to as "conventional technology 3").

It is known that the antibodies in immune response are immunoglobulin M (hereinafter, may be abbreviated as "IgM") which increases in patient blood in a relatively early phase of infection after a living body comes into contact with the antigen by infection and IgG which subsequently increases.

Here, a typical Ig is composed of light and heavy chains, but Camelids are known to produce heavy-chain antibodies not including light chains. A single domain including a variable region of a heavy-chain antibody is a natural single domain and functions as an antibody also by itself. Accordingly, it is called "single domain antibody" (hereinafter, may be abbreviated as "VHH antibody").

When the conformational epitope of a virus includes, for example, a hole-like structure like an enzyme pocket or a structural moiety of a split between domains, existing IgG cannot bind to such an epitope due to the size thereof. In contrast, the above-described VHH antibodies have a small molecular weight, one-tenth that of IgG antibody, and therefore can bind also to epitopes having the above-mentioned structure and can bind also to, for example, the surface of a virus particle modified with many sugar chains. Accordingly, the range of molecules that can be target molecules is widened.

Furthermore, the VHH also has excellent acid resistance and heat resistance and can be produced in *Escherichia coli,* yeast, or the like without need of being produced in cultured cells, unlike IgG. Accordingly, there are advantages of ease in mass production and also ease in purification. Furthermore, it is known that since the VHH antibody is composed of a single chain peptide, it has characteristics that the function can be easily modified using protein engineering technology or chemical modification technology and an antibody-drug conjugate (ADC) is easily produced (hereinafter, referred to as "conventional technology 4") .

[Non Patent Literature 1] Neutralizing nanobodies bind SARS-CoV-2 spike RBD and block interaction with ACE2. Nature Structural & Molecular Biology, 2020

[Non Patent Literature 2] Development of multi-specific humanized llama antibodies blocking SARS-CoV-2/ACE2 interaction with high affinity and avidity. Emerging Microbes & Infections, 2020, 9: 1034-1036

[Non Patent Literature 3] Structural Basis for Potent Neutralization of Betacoronaviruses by Single-Domain Camelid Antibodies. Cell, 2020, 181: 1004-1015

[Non Patent Literature 4] Identification of Human Single-Domain Antibodies against SARS-CoV-2. Cell Host & Microbe, 2020, 27: 891-898

The conventional technology 1 is a method for detecting a viral genetic material and is recognized to be most precise among virus tests that can be used at the moment and is excellent technology in terms of high accuracy. However, it takes time and labor to carry out the inspection, and there is a problem of being difficult to increase the number of PCR tests carried out to the number really needed.

The conventional technology 2 is excellent technology in terms that infection can be diagnosed in minutes without an expensive machine, training to master the skills required for inspectors, extensive effort required for inspection, and so on. Accordingly, if a reliable antigen testing method is established, the number of tests can be easily increased. In addition, since COVID-19 infection can be diagnosed at home or a medical site, introduction to a clinical site as soon as possible is required. However, there are problems that the cost for preparation of an antibody is high and that it is not easy to secure an amount thereof necessary for the test.

The conventional technology 3 is an excellent method in terms of being capable of comprehending the situation over time in the infection of CORVID-19 which is inferred to have a long incubation period from infection to onset in many cases and also has been reported to have a long clinical course in some cases such that after about one week from the onset, the symptoms worsen rapidly leading to severe pneumonia. However, even in such a serological method, since the test uses an antibody, there are the same problems as in the antigen test.

A typical example of the antibody that is used in the serological diagnosis is IgG, which is generally manufactured by purification from the serum of an animal administered with an antigen or by incorporating a gene fragment encoding the nucleotide sequence of the target IgG into a plasmid or the like, introducing the plasmid into a cultured cell, and culturing the cell. Accordingly, there are strong social demands for a molecule that functions as an antibody of which purification does not need time and labor.

The VHH has various advantages as described above but is difficult to acquire a VHH having a high affinity to the substrate. Accordingly, there are strong social demands for acquiring a VHH that has a high affinity to the substrate, is easily produced and purified, and can correspond to the substrate (nucleotide sequence of a virus) which is likely to mutate, in particular, a VHH that binds to SARS-CoV-2.

In addition, since IgG antibodies are intended for systemic administration via intravenous or subcutaneous administration, there is a problem that sufficient therapeutic effect is not expected compared to local administration. In contrast, a risk of antibody-dependent enhancement (ADE) by exposure to the whole body is also worried. Furthermore, regarding VHH-72-Fc, it has been reported that the VHH alone does not exhibit sufficient neutralizing activity, and risks of losing the physical superiority of a single domain antibody by fusion of Fc and also of antibody-dependent enhancement (ADE) via Fc are also worried.

From the above, in order to develop an agent showing excellent therapeutic effect against SARS-related coronaviruses, there are strong social demands for development of single domain antibodies that recognizes various epitopes.

Summary of the Invention

The present invention relates to a SARS-CoV-2-binding peptide comprising one or more structural domains comprising CDR3 consisting of an amino acid sequence of any of SEQ ID NOs: 1 to 9 or an amino acid sequence obtained by substituting one amino acid in the amino acid sequence with another amino acid.

In one aspect, the peptide is one selected from the group consisting of a VHH antibody, a heavy-chain antibody, and a VHH antibody multimer.

In another aspect, the VHH antibody multimer is a multimer formed having a plurality of the structural domains linked to each other.

In another aspect, the amino acid sequence represented by any of SEQ ID NOs: 1 to 9 has a KD of $3.6 \times 10^{-9}$ M or less for SARS-CoV-2.

In another aspect, the structural domain further comprises: CDR1 consisting of an amino acid sequence of any of SEQ ID NOs: 10 to 18 or an amino acid sequence obtained by substituting at least one amino acid in the amino acid sequence with another amino acid; and CDR2 consisting of an amino acid sequence of any of SEQ ID NOs: 19 to 27 or an amino acid sequence obtained by substituting at least one amino acid in the amino acid sequence with another amino acid.

In addition, another aspect of the present invention is a nucleic acid encoding the above-mentioned peptide.

In addition, another aspect of the present invention is a method for detecting SARS-CoV-2 in a sample comprising a step of contacting any of the above-described peptides with a test sample.

In addition, another aspect of the present invention is a kit for detecting SARS-CoV-2 comprising any of the above-described peptides.

In addition, another aspect of the present invention is a medicament comprising any of the above-described peptides.

In addition, another aspect of the present invention is use of any of the above-described peptides for manufacturing a medicament.

In addition, another aspect of the present invention is any of the above-described peptides for use as a medicament.

In addition, another aspect of the present invention is a method for preventing or treating SARS-CoV-2 infectious disease comprising administering any of the above-described peptide to a subject in need thereof.

Brief Description of Drawings

[Figure 1] Figure 1 is a graph showing change in the number of CDR3 clusters of anti-SARS-CoV-2 antibody candidates appeared after selection in each round.

[Figure 2] Figure 2 is a graph showing the results of evaluation by ELISA of the binding ability of the VHH clones selected by the selection.

[Figure 3] Figure 3 is graphs showing the binding characteristics of VHH clones by bio-layer interferometry.

[Figure 4] Figure 4 shows sensorgrams of competitive inhibition between the obtained VHH clones.

[Figure 5] Figure 5 is a graph showing inhibition rate against SARS-CoV-2 infection by a VHH clone.

[Figure 6] Figure 6 is graphs showing binding activities to SARS-CoV-2 variant strains.

[Figure 7] Figure 7 is graphs showing binding activities to SARS-CoV-2 variant strains of VHH-COVE6.

[Figure 8] Figure 8 is a graph showing binding activities to SARS-CoV-2 British/Nigeria variant strains.

[Figure 9] Figure 9 is graphs showing binding activities to SARS-CoV-2 California variant strain and India variant strain.

[Figure 10] Figure 10 is a graph showing competitive inhibition of each VHH clone with respect to the binding of RBD and ACE2.

[Figure 11] Figure 11 is SDS-PAGE showing the binding activity of VHH-COVE3 to RBD derived from India variant strain.

[Figure 12] Figure 12 is a graph showing neutralizing activities of VHH-COVE3 to SARS-CoV-2 alpha and delta variant strains.

[Figure 13] Figure 13 is a graph showing competitive inhibition of VHH-COVE3 with respect to the binding of RBD (L452R, E484Q) and ACE2.

[Figure 14] Figure 14 is a graph showing competitive inhibition of VHH-COVE3 with respect to the binding of RBD (L452R, T478K) and ACE2.

[Figure 15] Figure 15 is a graph showing neutralizing activity of VHH-COVE9 to SARS-CoV-2 alpha variant strain in an animal body.
[Figure 16] Figure 16 is SDS-PAGE showing binding activity of VHH-COVE3 to RBD derived from lambda variant strain.
[Figure 17] Figure 17 is a graph showing neutralizing activity of VHH-COVE3 to SARS-CoV-2 alpha variant strain in an animal body.
[Figure 18] Figure 18 is a graph showing neutralizing activity of VHH-COVE3 to SARS-CoV-2 delta variant strain in an animal body.

Detailed Description of the Invention

The present invention relates to providing a peptide that binds to SARS-CoV-2 and a usage thereof.
The present inventors studied to obtain peptides that bind to SARS-CoV-2 and as a result, succeeded in obtaining clones having a high reactivity with SARS-CoV-2 from a VHH antibody library containing CDR1 to CDR3 each having a specific number of amino acids in its construct by screening through a cDNA display method.
According to the present invention, it is possible to provide a SARS-CoV-2-binding peptide having a high reactivity that can contribute to creation of antibody drugs having strong neutralizing capacity and so on that cannot be obtained in known antibodies. The use of the peptide allows detection of SARS-CoV-2, i.e., rapid detection of new coronavirus infectious disease (COVID-19), which is SARS-CoV-2 infectious disease.
In addition, it is possible to provide a peptide that can be locally administered by an inhaler directly to the site of infection, the upper respiratory tract or lung, and can contribute to drug discovery of a respiratory infection therapeutic agent.
The SARS-CoV-2-binding peptide of the present invention (hereinafter, referred to as "peptide of the present invention") is a peptide that binds to SARS-CoV-2 and has one or more structural domains containing CDR3 consisting of an amino acid sequence of any of SEQ ID NOs: 1 to 9 or an amino acid sequence obtained by substituting at least one amino acid in the amino acid sequence with another amino acid.
Here, SARS-CoV-2 is a SARS-related coronavirus (COVID-19), which causes acute respiratory disease, and a single-stranded positive-sense RNA virus whose viral genome consists of 29,903 nucleotides. The peptide of the present invention is a peptide that binds to SARS-CoV-2, for the details, a peptide that binds to the S1 subunit of SARS-CoV-2 spike protein.
The peptide of the present invention is a peptide having an ability of potentially binding to an epitope and corresponds typically to an antibody or an antibody fragment. The term "antibody" includes IgA, IgD, IgE, IgG, and IgM which are generated by antibody-forming cells responsible for humoral immunity, a heavy-chain antibody, a single-chain antibody, an ScFv, a single domain antibody containing the CDR3 sequence of a heavy-chain antibody (hereinafter, may be referred to as "VHH" or "VHH antibody"), an aptamer that can bind to a foreign substance such as a pathogen entered into the body, and those obtained by modification or alteration of parts of them.
In the present specification, the "CDR3 cluster" is defined as a group of 80% identity (the threshold of clustering) for the amino acid sequence of CDR3, which is one of complementarity determining regions, determined using CD-HIT (Cluster Database at High Identity with Tolerance) or another sequence clustering program.
The peptide of the present invention contains at least three complementarity determining regions (hereinafter, may be referred to as "CDRs") and a plurality of framework regions. The CDR is also referred to as a hypervariable region and is a region containing a sequence-variable antigen-recognition site or a random sequence region. The positional relationship among the CDRs is in the order of CDR1, CDR2, and CDR3 from the N-terminal side. The framework region (hereinafter, may be referred to as "FR") is a region excluding the complementarity determining region in the variable region of an antibody molecule, i.e., a region showing high conservation, and may be located at both the 5' side and the 3' side of the CDR1 to CDR3 referring to the frames.
In the peptide of the present invention, among CDR1 to CDR3, CDR3 consists of any of the sequences shown in Table 1 below or an amino acid sequence obtained by substituting at least one amino acid in any of these amino acid sequences with another amino acid. Here, although the position of the amino acid to be substituted and the type of the amino acid after the substitution are not limited as long as the dissociation constant of the amino acid sequence to the antigen does not exceed 100 nM, one substitution is preferable.

[Table 1]

| | CDR3 sequence | Number of CDR3 residues | SEQ ID NO: |
|---|---|---|---|
| VHH-COVE1 | LLRRYNYGFTFDNY | 14 | 1 |
| VHH-COVE2 | FGGSDFLMDY | 10 | 2 |

(continued)

|  | CDR3 sequence | Number of CDR3 residues | SEQ ID NO: |
|---|---|---|---|
| VHH-COVE3 | AWSDYEYLEV | 10 | 3 |
| VHH-COVE4 | TPWYSASHTY | 10 | 4 |
| VHH-COVE5 | VTWLRGDY | 8 | 5 |
| VHH-COVE6 | ITTGSPLLGGGMDF | 14 | 6 |
| VHH-COVE7 | VFPSGGDY | 8 | 7 |
| VHH-COVE8 | VGLFGIQASDY | 11 | 8 |
| VHH-COVE9 | PGVVTGSYDVRNY | 13 | 9 |

The peptide including such a CDR3 is a clone having a high reactivity with SARS-CoV-2, obtained by screening through a cDNA display method in Examples described below, that is, a SARS-CoV-2-binding peptide (anti-SARS-CoV-2 antibody (CoVHH)).

The binding ability to SARS-CoV-2 can be evaluated by a method known to those skilled in the art. Specifically, as shown in Examples described below, the binding ability can be evaluated by determining the dissociation constant KD by bio-layer interferometry. The binding ability can also be evaluated by a method, for example, antigen-immobilized ELISA, immunochromatography, isothermal titration calorimetry, surface plasmon resonance analysis, or the like. The dissociation constant KD in the present invention was determined by bio-layer interferometry.

Among the VHH-COVE1 to VHH-COVE9, in terms of neutralizing activity to SARS-CoV-2, VHH-COVE2, VHH-COVE5, VHH-COVE8, and VHH-COVE9 are preferable.

In the structural domains of the peptide of the present invention, for example, the CDR1 may be the amino acid sequence of any of SEQ ID NOs: 10 to 18, and the CDR2 may be the amino acid sequence of any of SEQ ID NOs: 19 to 27. These amino acid sequences may be those obtained by substituting at least one amino acid in these amino acid sequences with another amino acid.

For example, the amino acid sequences of three complementarity determining regions in the structural domains of VHH-COVE1 to VHH-COVE9 are as follows.

[Table 2]

|  | CDR1 | CDR2 | CDR3 |
|---|---|---|---|
| VHH-COVE1 | SEQ ID NO: 10 | SEQ ID NO: 19 | SEQ ID NO: 1 |
| VHH-COVE2 | SEQ ID NO: 11 | SEQ ID NO: 20 | SEQ ID NO: 2 |
| VHH-COVE3 | SEQ ID NO: 12 | SEQ ID NO: 21 | SEQ ID NO: 3 |
| VHH-COVE4 | SEQ ID NO: 13 | SEQ ID NO: 22 | SEQ ID NO: 4 |
| VHH-COVE5 | SEQ ID NO: 14 | SEQ ID NO: 23 | SEQ ID NO: 5 |
| VHH-COVE6 | SEQ ID NO: 15 | SEQ ID NO: 24 | SEQ ID NO: 6 |
| VHH-COVE7 | SEQ ID NO: 16 | SEQ ID NO: 25 | SEQ ID NO: 7 |
| VHH-COVE8 | SEQ ID NO: 17 | SEQ ID NO: 26 | SEQ ID NO: 8 |
| VHH-COVE9 | SEQ ID NO: 18 | SEQ ID NO: 27 | SEQ ID NO: 9 |

FR1 may be the amino acid sequences of SEQ ID NOs: 28 to 34, and FR2 may be the amino acid sequences of SEQ ID NOs: 35 to 42. FR3 may be the amino acid sequences of SEQ ID NOs: 43 to 50, and FR4 may be the amino acid sequences specified by the nucleotide sequences of SEQ ID NOs: 51 to 53.

In addition, it is preferable that FR1 to FR4 include amino acid sequences having 80% or more identity with the amino acid sequences of SEQ ID NOs: 28 to 53. Here, the amino acid sequences having 80% or more identity with the amino acid sequences of SEQ ID NOs: 28 to 53 are more preferably sequences having 85% or more identity, further more preferably 90% or more identity, with the amino acid sequences of SEQ ID NOs: 28 to 53. FR1 to FR4 are further more preferably framework regions consisting of the amino acid sequences having 99% or more identity.

Here, the identity of an amino acid sequence refers to the rate (%) of the number of positions where the same amino acid residue is present in both two amino acid sequences that are aligned relative to the number of full-length amino acid residues. Specifically, it can be calculated by analysis using, for example, the Basic Local Alignment Search Tool (BLAST) of the National Center for Biotechnology Information (NCBI).

Examples of the peptide including the sequence of CDR3 of the present invention include that linked in the order of FR1-CDR1-FR2-CDR2-FR3-CDR3-FR4 from the N-terminal side toward the C-terminal side. The amino acid sequences of these regions are represented by SEQ ID NOs: 1 to 53 in Sequence Listing as described above, and the amino acid sequences of the regions of the above-described VHH-COVE1 to VHH-COVE9 are represented by SEQ ID NO: 65 (VHH-COVE1), SEQ ID NO: 66 (VHH-COVE2), SEQ ID NO: 67 (VHH-COVE3), SEQ ID NO: 68 (VHH-COVE4), SEQ ID NO: 69 (VHH-COVE5), SEQ ID NO: 70 (VHH-COVE6), SEQ ID NO: 71 (VHH-COVE7), SEQ ID NO: 72 (VHH-COVE8), and SEQ ID NO: 73 (VHH-COVE9).

The peptide of the present invention is not limited in its form as long as at least one of the above-mentioned structural domains is included and may be not only a variable region fragment (also referred to as a nanobody) such as a VHH antibody but also a multimer, for example, a dimer, obtained by linking a single-chain antibody, a heavy-chain antibody, or a variable region fragment with a peptide linker or the like.

The method for producing the peptide of the present invention is not particularly limited and can be easily produced by technology known in the art. For example, the peptide can be produced by a combination of peptide solid-phase synthesis and native chemical ligation (NCL) or by genetic engineering, but preferred is a method in which a nucleic acid encoding the peptide of the present invention is incorporated into an appropriate vector (e.g., plasmid) and the vector is introduced into a host cell to produce the peptide as a recombinant peptide.

Here, as the expression plasmid of the peptide of the present invention, plasmids suitable for various host cells can be used. For example, it is possible to use plasmids derived from *Escherichia coli,* such as pBR322, pBR325, pUC12, and pUC13; vectors derived from *Bacillus subtilis,* such as pUB110, pTP5, and pC194; vectors derived from yeast, such as pSH19 and pSH15; bacteriophages, such as λ phage; virus vectors, such as adenovirus, adeno-associated virus, lentivirus, vaccinia virus, and baculovirus; and vectors obtained by appropriately modifying them.

These expression plasmids include initiation sites of replication, selection markers, and promoters suitable for the respective plasmids and may include enhancers, transcription termination sequences (terminators), liposome-binding sites, polyadenylation signals, and so on as needed. Furthermore, the expression plasmids may be inserted with nucleotide sequences for expression in fusion with a FLAG tag, His tag, HA tag, or GST tag for the purpose of facilitating purification of the expressed polypeptides.

The bacteria that generate the peptide of the present invention can be produced by a desired method, for example, by introducing the above-mentioned expression plasmid into a desired bacterium by electroporation. Examples of the host cell to be used for the generation of a recombinant peptide include cells such as *Escherichia coli, Bacillus subtilis, Corynebacterium,* various molds, animal cells, and plant cells, and baculovirus/insect cells or yeast cells. Among these cells, suitably used are *Corynebacterium* and *Bacillus subtilis,* and *Bacillus subtilis,* which shows high productivity, is more preferable.

When the expressed peptide of the present invention is extracted from the cultured bacteria or cultured cells, after the culture, the bacterial cells or cultured cells are collected by a known method and are suspended in an appropriate buffer solution. The bacteria or cells are broken by ultrasonication, lysozyme treatment, and/or freezing and thawing, and the soluble extract is then acquired by centrifugation or filtration. The target peptide can be acquired from the resulting extraction liquid by appropriately combining known separation and purification methods.

As the known separation and purification, for example, a method utilizing solubility, such as salting out and solvent precipitation; a method mainly utilizing the difference in molecular weight, such as dialysis, ultrafiltration, gel filtration, and SDS-PAGE; a method utilizing the difference in charge, such as ion exchange chromatography; a method utilizing specific affinity, such as affinity chromatography; a method utilizing the difference in hydrophobicity, such as reversed phase high performance liquid chromatography; or a method utilizing the difference in isoelectric point, such as isoelectric point electrophoresis, can be used.

The peptide of the present invention was found by selection of VHH antibody candidate sequences using a cDNA display method, as shown in Examples described later. Acquisition of antibodies when this method is used will now be described.

A SARS-CoV-2 (2019-nCoV) spike S1-His recombinant protein (hereinafter, abbreviated as "SARS-CoV-2 spike S1 protein", Sino Biological, Inc.) was used as a target molecule for screening. Two gene fragments of CDR1 and CDR2 regions or CDR3 region are amplified by PCR with VHH specific primers using alpaca-derived naive VHH library genes provided by RePHAGEN Co., Ltd. as templates and are extended by performing extension PCR using overlap PCR primers to produce a full-length VHH-encoding DNA library.

Examples of the VHH specific primer used here include SEQ ID NOs: 54 and 55 in Sequence Listing. Examples of the overlap PCR primer include SEQ ID NOs: 56 and 57 in Sequence Listing. The DNA fragment constituting the library preferably includes a T7 promoter, an omega (Ω) enhancer, a Kozak consensus sequence, a VHH gene, a His tag, and a linker hybridization region (Y tag).

The thus obtained library (PCR product) is purified, and transcription is subsequently performed. The purification of the library can be performed using a commercially available kit, for example, Agencourt AMPure XP (Beckman Coulter, Inc.). The transcription can use a commercially available kit, for example, T7 RiboMAX Express Large Scale RNA Production System (Promega Corporation). Subsequently, RNA is quantitatively measured using, for example, NanoPad DS-11FX (DeNovix Inc.).

The obtained mRNA is linked to a puromycin linker including a photocrosslinked nucleotide of cnvK or an analogue thereof by irradiation with light of a desired wavelength for desired time, e.g., light of 330 to 370 nm for 1 to 10 minutes to obtain a mRNA-linker conjugate. Cell-free translation using the obtained mRNA-linker conjugate is performed to synthesize a mRNA-VHH conjugate from the mRNA-linker conjugate. This cell-free translation can use a commercial product such as Rabbit Reticulocyte Lysate System, Nuclease Treated (Promega Corporation).

Subsequently, the obtained mRNA-VHH conjugate is immobilized on magnetic beads. As the beads, for example, Dynabeads My One Streptavidin C1 (Thermo Fisher Scientific) can be used. The mRNA-VHH conjugate not immobilized on the magnetic beads is removed, and reverse transcription reaction is performed to synthesize a cDNA-VHH conjugate.

Subsequently, the cDNA-VHH conjugate immobilized on the magnetic beads is eluted by, for example, adding a His-tag wash buffer containing RNase T1, and His-tag purification is performed to obtain a purified cDNA-VHH conjugate.

In order to immobilize a desired target molecule, e.g., SARS-CoV-2 spike S1 protein on, for example, the above-described magnetic beads, the target molecule is biotinylated in accordance with a usual method. Since the thus biotinylated target protein binds to streptavidin immobilized on the beads, the target molecule can be immobilized on the beads. The protein concentration of the solution is measured before and after the immobilization, and the band intensity ratios on SDS-PAGE are compared to verify the yield of the immobilization reaction.

Subsequently, using the thus obtained cDNA-VHH conjugate (cDNA display molecule) and beads on which a target molecule, e.g., SARS-CoV-2 spike S1 protein is immobilized, in vitro selection is performed. The sequence of resulting VHH is preferably converged by repeating this selection several times, and 3 to 6 rounds repetition is preferable for increasing the selection efficiency.

In this selection, a cDNA display method is used, and it is preferable to control the amount of the mRNA-linker conjugate to be used in each round and to perform preselection for removing non-specific adsorbates as needed. The eluate to be used in the elution at each round and the number of elution times can be appropriately selected. For example, the number of elution times in round 1 may be different from those in the subsequent rounds, and multiple elution buffers may be used in each round. The obtained eluate is purified using, for example, Agencourt AMPure XP (Beckman Coulter, Inc.) to obtain a purified product.

Subsequently, the purified product obtained by selection is subjected to PCR for amplification to obtain a PCR amplification product. The obtained PCR amplification product is immobilized, for example, on the above-mentioned magnetic beads as in above to obtain FITC-labeled beads for FACS sorting. The region of the target protein-immobilized beads is identified. Sorting is performed under optimum fractionation conditions to collect a desired number of particles, for example, 500,000 particles each, and purification is performed as above. The obtained particles are amplified using, for example, PrimeSTAR HS DNA Polymerase and purified as above.

Subsequently, sequence analysis utilizing a next generation sequencer (NGS) is performed to verify in detail the degree of convergence of the DNA library by in vitro selection cycle. Sequence samples can be prepared, for example, in accordance with 2-step PCR Amplicon Library Preparation provided by Illumina, Inc. Amplicon PCR using the PCR product after each selection as the template can be performed using desired primers, for example, primers of SEQ ID NOs: 60 and 61.

The PCR product thus obtained by Amplicon PCR is purified as described above, and Index PCR is then performed. The obtained PCR product is purified as described above, and the size of the PCR product is then verified by gel electrophoresis. Subsequently, the PCR product is quantitatively measured using, for example, Nanodrop, the molar concentration is calculated, for example, in accordance with the equation (1) below, and based on the obtained value, the concentration is adjusted to a desired concentration, for example, 4 nM by dilution with NFW.
(Math 1)

$$\text{DNA concentration [ng/}\mu\text{L]}/(660 \text{ [g/mol]} \times 550 \text{ [bp]}) \times 10^6 = \text{DNA concentration [nM]} \quad (1)$$

The library concentration is controlled in accordance with a recommended protocol to adjust the concentrations of the denatured and diluted sample library and the control to desired final concentrations. Subsequently, sequencing can be performed using, for example, MiSeq (Illumina, Inc.) and MiSeq Reagent Nano Kit v2500 cycle (Illumina, Inc.).

Continuously, the obtained demultiplexed sequence data are subjected to analysis to extract nucleotide sequence regions encoding a VHH antibody, and the nucleotide regions are translated into amino acid sequences. Based on these translation data, the number of amino acid sequences that perfectly agree with each amino acid sequence is counted. Then, one of complementarity determining regions, for example, the sequence of CDR3 is used as a target, and the number of clones is counted with a desired similarity of 80% or more as the same cluster using, for example, the sequence clustering program CD-HIT to determine the number of clusters in each round.

Clones that are thought to be non-specific binding based on, for example, comparison of data of FACS sorting are removed, and VHH antibody candidate sequences that bind to a target molecule are then selected. The selected VHH antibody candidate sequences are amplified by PCR using desired primers, for example, primers of SEQ ID NOs: 63 and 64, and the obtained PCR amplification product is purified to prepare a gene for producing an VHH antibody. A polyclonal VHH antibody is produced from the obtained gene by a cell-free translation system. The antibody titer of the obtained polyclonal VHH antibody can be evaluated by, for example, ELISA in accordance with a usual method.

In protein expression of a VHH clone including a CDR3 sequence that showed the binding in ELISA evaluation, a gene of each VHH is prepared from the DNA library of R5 in the above-described in vitro screening and is introduced into an expression plasmid. The DNA of a target fragment is cut out using a restriction enzyme and is ligated to an expression plasmid to prepare a recombinant plasmid, and a desired bacterium, for example, *Escherichia coli* is transformed. The recombinant plasmid is extracted from the transformed *Escherichia coli* to obtain an expression plasmid. The obtained expression plasmid is introduced into a desired bacterium, the obtained antibody-producing bacterium is pre-cultured in a medium containing an antibiotic and is then main-cultured, and the culture supernatant is collected. The generation of VHH can be verified by, for example, gel electrophoresis. The collected culture supernatant is subjected to His-tag purification to obtain a VHH antibody sample.

The peptide of the present invention binds to SARS-CoV-2, which can verify whether SARS-CoV-2 is present or not in a test sample that contains or may contain SARS-CoV-2 by contacting the peptide with the sample.

Specifically, the detection of SARS-CoV-2 using the peptide of the present invention includes a step of contacting the peptide of the present invention with a test sample to form a conjugate of the peptide of the present invention and SARS-CoV-2 in the test sample and a step of detecting SARS-CoV-2 in the conjugate.

In detection of a virus-specific antibody in serum, a part of a SARS-CoV-2 antigen is added and bound to an immobilized anti-SARS-CoV-2 antibody (e.g., serum antibody) contained in a test sample (serum), and the peptide of the present invention can also be used as a peptide for verifying the presence of the SARS-CoV-2 antigen in the conjugate state.

Examples of the test sample include biological samples such as a tracheal swab, a nasal swab, a pharyngeal swab, a nasal lavage fluid, a nasal aspirate, a nasal discharge, saliva, sputum, blood, serum, urine, feces, a tissue, a cell, and a tissue or cell debris, and samples taken from solid surfaces, e.g., a doorknob and a toilet bowl, to which viruses may adhere. The peptide of the present invention may be immobilized to a solid phase or may not be immobilized to a solid phase.

The step of detecting SARS-CoV-2 in the conjugate can be carried out by, for example, reacting the conjugate with an anti-SARS-CoV-2 antibody that recognizes an epitope different from the peptide of the present invention in the conjugate. Alternatively, SARS-CoV-2 in the conjugate may be detected in a liquid phase by a homogeneous assay.

The peptide of the present invention can be a component of a kit for detecting SARS-CoV-2. The kit can be used as a diagnostic agent for SARS-CoV-2 infectious disease (COVID-19) or as a tool for developing a therapeutic agent for SARS-CoV-2 infectious disease.

The detection kit can include, in addition to the SARS-CoV-2-binding peptide of the present invention, reagents and tools necessary for the detection, for example, an antibody, a solid-phase carrier, a buffer solution, an enzyme reaction stopping solution, and a microplate reader.

In the detection kit, the peptide of the present invention may be immobilized on a solid phase. Examples of the solid phase include beads, membrane, a side face or bottom face of a reaction container, a plate-shaped substrate such as slide glass, and a substrate having wells such as an immunoplate (hereinafter, referred to as "well substrate"), and the peptide of the present invention is immobilized directly or indirectly to them.

When the peptide of the present invention binds to the S1 subunit of SARS-CoV-2 and inhibits the binding of viruses to cells, the peptide of the present invention can be used as a medicament for preventing or treating SARS-CoV-2 infectious disease by administration to a mammal. Examples of the mammal include a human, a mouse, a rat, a hamster, a guinea pig, a rabbit, a cat, a dog, a monkey, a cow, a horse, and a pig, and the mammal is preferably a human.

When the peptide of the present invention is used as a medicament, the dosage form may be oral or parenteral, and the peptide can be appropriately used in combination with well-known pharmaceutically acceptable excipient, diluent, and so on. Examples of the parenteral administration include intravenous, subcutaneous, intradermal, and intramuscular administration and administration to mucous membrane by spraying or the like.

The administration may be performed by appropriately adjusting the content of the peptide of the present invention contained in a medicament and administering an effective dose one to several times a week.

Regarding the above-described embodiments, the present invention further discloses the following aspects:

<1> A SARS-CoV-2-binding peptide comprising one or more structural domains comprising CDR3 consisting of an amino acid sequence of any of SEQ ID NOs: 1 to 9 or an amino acid sequence obtained by substituting at least one amino acid in the amino acid sequence with another amino acid;

<2> The peptide according to <1>, wherein the SARS-CoV-2-binding peptide is one selected from the group consisting of a VHH antibody, a heavy-chain antibody, and a VHH antibody multimer;

<3> The peptide according to claim 2, wherein the VHH antibody multimer is a multimer having a plurality of the structural domains linked to each other;

<4> The peptide according to any of <1> to <3>, wherein the amino acid sequence of any of SEQ ID NOs: 1 to 9 has a KD of $3.6 \times 10^{-9}$ M or less for SARS-CoV-2;

<5> The peptide according to any of <1> to <4>, wherein the structural domain further comprises: CDR1 consisting of an amino acid sequence of any of SEQ ID NOs: 10 to 18 or an amino acid sequence obtained by substituting at least one amino acid in the amino acid sequence with another amino acid; and CDR2 consisting of an amino acid sequence of any of SEQ ID NOs: 19 to 27 or an amino acid sequence obtained by substituting at least one amino acid in the amino acid sequence with another amino acid;

<6> The peptide according to <5>, selected from 1) to 9), the peptide comprising one or more structural domains comprising each of CDR1, CDR2, and CDR3 consisting of an amino acid sequence of SEQ ID NOs: 1 to 27 shown in Table 2 or an amino acid sequence obtained by substituting at least one amino acid in the amino acid sequence with another amino acid;

<7> A nucleic acid encoding the peptide according to <1>;

<8> A method for detecting SARS-CoV-2 in a sample, comprising a step of contacting the peptide according to any of <1> to <6> with a test sample;

<9> A kit for detecting SARS-CoV-2 comprising the peptide according to any of <1> to <6>;

<10> A medicament comprising the peptide according to any of <1> to <6>;

<11> The medicament according to <10> for preventing or treating SARS-CoV-2 infectious disease;

<12> Use of the peptide according to any of <1> to <6> for manufacturing a medicament;

<13> The use according to <12>, wherein the medicament is a medicament for preventing or treating SARS-CoV-2 infectious disease;

<14> The peptide according to any of <1> to <6> for use as a medicament;

<15> The peptide according to <14>, wherein the medicament is a medicament for preventing or treating SARS-CoV-2 infectious disease; and

<16> A method for preventing or treating SARS-CoV-2 infectious disease comprising administering the peptide according to any of <1> to <6> to a subject in need thereof.

Examples

(Example 1) Screening for anti-SARS-CoV-2 single domain antibody

<Material and method>

1. Target molecule of screening

As a target molecule, SARS-CoV-2 (2019-nCoV) spike S1-His recombinant protein (hereinafter, abbreviated as "SARS-CoV-2 spike S1 protein", Sino Biological, Inc.) was used.

2. Synthesis of cDNA display

(1) Production of DNA library encoding variable region fragment of full-length alpaca-derived antibody (single Variable domain of the Heavy chain of a Heavy-chain antibody: VHH)

Two gene fragments of CDR1 and CDR2 regions or CDR3 region were amplified by PCR with VHH specific primers (SEQ ID NOs: 54 and 55) using alpaca-derived naive VHH library genes provided by RePHAGEN Co., Ltd. as templates and were then extended by performing extension PCR using overlap PCR primers (SEQ ID NOs: 56 and 57) for preparing a DNA fragment consisting of a T7 promoter, an omega (ω) enhancer, a Kozak consensus sequence, a VHH gene, a His tag, and a linker hybridization region (Y tag) to produce a full-length VHH-encoding DNA library.

[Table 3]

| Primer name | Sequence | SEQ ID NO: |
|---|---|---|
| VHH-SPECIFIC PRIMER | 5'-GAT CCC GCG AAA TTA ATA CGA CTC ACT ATA GGG GAA GTA TTT TTA CAA CAA TTA CCA ACA ACA ACA ACA AAC AAC AAC AAC ATT ACA TTT TAC ATT CTA CAA CTA CAA GCC ACC ATG-3' | 54 |
| | 5'-TTT CCA CGC CGC CCC CCG TCC TGC TTC CGC CGT GAT GAT GAT GAT GAT GGC TGC CTC CCC C-3' | 55 |
| Overlap PCR primer | 5'-GAT CCC GCG AAA TTA ATA CGA CTC ACT ATA GGG GAA GTA TTT TTA CAA CAA TTA CCA ACA ACA ACA ACA AAC AAC AAC AAC ATT ACA TTT TAC ATT CTA CAA CTA CAA GCC ACC ATG-3' | 56 |
| | 5'-TTT CCA CGC CGC CCC CCG TCC TGC TTC CGC CGT GAT GAT GAT GAT GAT GGC TGC CTC CCC C-3' | 57 |

The PCR product was purified using Agencourt AMPure XP (Beckman Coulter, Inc.), and beads were added at 1.8 μL per μL of the PCR product. The mixture was mixed by pipetting and was then left to stand for 5 minutes. After leaving to stand on a magnetic plate until becoming transparent, the supernatant was removed. After addition of 1.4 mL of 70% ethanol and leaving to stand for 30 seconds, the supernatant was removed. This ethanol washing was performed three times. After the supernatant was removed, the tube was left to stand on a magnetic plate for 3 minutes, and the beads were air dried. The tube was unloaded from the magnetic plate, 100 μL of nuclease-free water was added to the tube, and the beads were suspended by pipetting, followed by leaving to stand for 5 minutes. The tube was left to stand on a magnetic plate for 2 minutes, and the supernatant was collected.

(2) In vitro transcription

T7 RiboMAX Express Large Scale RNA Production System (Promega Corporation) was used. A mixture of 25 μL of RiboMAX (TM) Express T7 2x Buffer, 20 pmol of the PCR product, and 5 μL of Enzyme Mix was incubated at 37°C for 30 minutes. Subsequently, 5 μL of RQ1 RNase-Free DNAse was added thereto, followed by incubation at 37°C for 15 minutes. The transcription product was purified using RNAClean XP (Beckman Coulter, Inc.). Beads were added to the tube at 1.8 μL per μL of the transcription product in the tube, followed by mixing by pipetting and then leaving to stand for 5 minutes. After leaving to stand on a magnetic plate, the supernatant was removed. After addition of 200 μL of 70% ethanol and leaving to stand for 30 seconds, the supernatant was removed. This ethanol washing was performed three times. After the supernatant was removed, the tube was left to stand on a magnetic plate for 10 minutes, and the beads were air dried. The tube was unloaded from the magnetic plate, 20 μL of nuclease-free water was added to the tube, and the beads were suspended by pipetting, followed by leaving to stand for 5 minutes. The tube was left to stand on a magnetic plate for 1 minute, and the supernatant was then collected. After purification, RNA was quantitatively measured by NanoPad DS-11FX (DeNovix Inc.).

(3) Linking of mRNA and puromycin linker

A mixture of 4 μL of 0.25 M Tris-HCl (pH 7.5), 4 μL of 1 M NaCl, 20 pmol of mRNA, and 20 pmol of cnvK ribo G linker was adjusted to 20 μL with nuclease-free water to prepare a reaction solution. Annealing was performed using ProFlex PCR System (Life Technologies Corporation) under conditions of incubation at 90°C for 2 minutes, at 70°C for 1 minute, at 25°C for 30 seconds, and at 4°C. The Ramp rate was set to 0.1°C/sec. Subsequently, light having a wavelength of 365 nm was irradiated for 5 minutes using Handheld UV Lamp (6 W, UVGL-58, measurement wavelength: 254/365 nm, 100 V, Analytik Jena US, an Endress+Hauser Company). The mRNA-linker conjugate was shielded from light and ice-cooled until use.

(4) Cell-free translation

In synthesis of a mRNA-VHH conjugate from the mRNA-linker conjugate, Rabbit Reticulocyte Lysate System, Nuclease Treated (Promega Corporation) was used. A reaction solution was prepared by mixing 10.5 μL of nuclease-free water, 15 μL of 20x Translation mix, 525 μL of rabbit reticulocyte lysate, 15 μL of RNasin (TM) Ribonuclease Inhibitor (40 U/μL) (Promega Corporation), and 9 μL of the mRNA-linker conjugate and was incubated at 37°C for 15 minutes, and 36 μL of IW formation buffer (3 M KCl, 1 M MgCl$_2$) mixture solution was added thereto, followed by further reaction at 37°C for 20 minutes to synthesize a mRNA-VHH conjugate.

(5) Immobilization to streptavidin magnetic beads

A suspension was prepared by adding 60 μL of 2x binding buffer (20 mM Tris-HCl, 2M NaCl, 0.2% Tween 20, 2 mM EDTA, pH 8) to 60 μL of Dynabeads My One Streptavidin C1 (Thermo Fisher Scientific) and pipetting for 1 minute. The suspension was left to stand on a magnetic plate for 1 minute, and the supernatant was discarded. This washing was performed twice. The washed streptavidin magnetic beads were mixed with 75 μL of the mRNA-VHH conjugate and 75 μL of 2x binding buffer, and the mixture was incubated at room temperature for 30 minutes. After leaving to stand on a magnetic plate for 1 minute, the supernatant was removed. After addition of 200 μL of binding buffer (10 mM Tris-HCl, 1 M NaCl, 0.1% Tween 20, 1 mM EDTA, pH 8) and pipetting for 1 minute, the supernatant was removed. This washing was performed twice.

(6) Reverse transcription reaction

A reaction solution was prepared by mixing 55.5 μL of nuclease-free water, 1.5 μL of 5x ReverTra Ace Buffer (Toyobo Life Science), 3 μL of 25 mM dNTP Mix, and 1.5 μL of ReverTra Ace (100 U/μL) (Toyobo Life Science). The mRNA-VHH conjugate immobilized on the streptavidin magnetic beads was added to the reaction solution, and reverse transcription reaction was performed by incubation at 42°C for 30 minutes to synthesize a cDNA-VHH conjugate.

(7) Cutting out from magnetic beads

His-tag wash buffer (20 mM Sodium phosphate, 500 mM NaCl, 5 mM Imidazole, 0.05% Tween 20, pH 7.4) was added to the cDNA-VHH conjugate immobilized on the streptavidin magnetic beads, followed by pipetting for 1 minute to prepare a suspension. The suspension was left to stand on a magnetic plate for 1 minute, and the supernatant was discarded. Subsequently, 30 μL of His-tag wash buffer containing 10 U of RNase T1 (Thermo Fisher Scientific) was added thereto, and a suspension was prepared by pipetting for 1 minute and was then left to stand at 37°C for 15 minutes to elute the cDNA-VHH conjugate.

(8) His tag purification

30 μL of His Mag Sepharose Ni Beads (GE Healthcare) were left to stand on a magnetic plate for 1 minute, the supernatant was discarded, and the beads were then re-suspended in His-tag wash buffer. This washing operation was performed twice. The eluate of the cDNA-peptide conjugate and the His Mag Sepharose Ni Beads suspension were mixed, incubated at room temperature for 30 minutes, and then left to stand on a magnetic plate for 1 minute, and the supernatant was discarded. A suspension was prepared by addition of 200 μL of His-tag wash buffer and pipetting for 1 minute. The suspension was left to stand on a magnetic plate for 1 minute, and the supernatant was discarded. This washing operation was performed twice. After addition of 10 μL of His-tag elution buffer (20 mM Sodium phosphate, 500 mM NaCl, 250 mM Imidazole, 0.05% Tween 20, pH 7.4), incubation was performed at room temperature for 15 minutes to elute the cDNA-VHH conjugate.

3. Selection

(1) Immobilization of target molecule on magnetic beads

The SARS-CoV-2 spike S1 protein as a target molecule was immobilized using Dynabeads My One Streptavidin C1 by the following procedure. First, 100 μL of 1x PBS buffer was added to 25 μg of the SARS-CoV-2 spike S1 protein, and 6.5 μL of 1 mM of EZ-Link (TM) Sulfo-NHS-SS-Biotin solution was then added to the solution, followed by incubation at room temperature for 30 minutes. The reaction solution was purified by using Zeba (TM) Spin Desalting Column (Thermo Fisher Scientific) to collect 116 μL of biotinylated SARS-CoV-2 spike S1 protein.
20 μL of Dynabeads My One Streptavidin C1 were put in an Eppendorf tube and left to stand on a magnetic plate for 1 minute, and the supernatant was then removed. The beads were re-suspended in 100 μL of 1x binding buffer (10 mM Tris-HCl, 1 M NaCl, 0.1% Tween 20, 1 mM EDTA, pH 8). After pipetting for 1 minute, the suspension was left to stand on a magnetic plate for 1 minute, and the supernatant was removed. 25 μL of the biotinylated SARS-CoV-2 spike S1 protein solution was added to the resultant, and the mixture was stirred at room temperature for 30 minutes, left to stand on a magnetic plate for 1 minute, and then washed with 100 μL of 1x binding buffer and 100 μL of PBST sequentially, followed by addition of 100 μL of PBST to obtain a SARS-CoV-2 spike S1 protein-immobilized beads solution. The yield of the immobilization reaction was estimated by measuring the protein concentration of the solution before and after the immobilization using NanoPad DS-11FX (DeNovix Inc.) and comparing the band intensity ratios on SDS-PAGE.

(2) In vitro selection

In vitro selection was repeated from round 1 to round 5 (R1 to R5) using the cDNA-VHH conjugate (cDNA display molecule) synthesized in 2-(8) of the above and the SARS-CoV-2 spike S1 protein-immobilized beads prepared in 3-(1) of the above under the conditions shown in Table 4 below by the following procedure. In cDNA display synthesis, a mRNA/linker ligation product was used in an amount of 192 pmol in (R1), 12 pmol in R2, and 6 pmol in R3, R4, and R5. In (R2) to (R5), preselection was performed in order to remove non-specific adsorbate. That is, the prepared cDNA display molecule was diluted with PBST to 100 μL and was allowed to bind to streptavidin-bound magnetic beads washed with 1x binding buffer in advance, followed by stirring at room temperature for 1 to 2 hours, then leaving to stand on a magnetic stand for 2 minutes, and collecting the supernatant. This operation was performed once or twice, and the obtained supernatant was diluted with PBST to 1 μM in (R1) and 100 nM in (R2) to (R4) with respect to the immobilized amount of the SARS-CoV-2 spike S1 protein, and was then allowed to bind to the SARS-CoV-2 spike S1-immobilized beads, followed by stirring at room temperature for 1 to 2 hours. After leaving to stand on a magnetic stand for 2 minutes, the supernatant was removed, and the magnetic beads were washed with 100 μL of PBST.

The washing operation was repeated once to three times, 40 μL of 10 mM TCEP buffer (pH 7 to 8), 10 mM NaOH solution, 1% SDS-containing PBST solution, or 20 μL of 1 μM SARS-CoV-2 spike S1 protein solution was then added as an elution buffer, incubation was performed at room temperature for 5 to 30 minutes, and the eluate was collected. The number of elution times at each round was four in (R1) and three in (R2) to (R5). In (R1) and (R2), the first and second elutions were performed with TCEP buffer, the third elution was performed with NaOH solution, and fourth elution in (R1) was performed with 1% SDS-containing PBST. In (R3) to (R5), the first and second elutions were performed with the SARS-CoV-2 spike S1 protein solution, and the third elution was performed with TCEP buffer. The obtained eluates were purified using Agencourt AMPure XP (Beckman Coulter, Inc.) in accordance with the manufacturer's instructions.

[Table 4]

| Round | mRNA/linker-ligation product (cDNA display) | Pre-selection | Time of washes |
|---|---|---|---|
| 1 | 192 pmol | None | Three times |
| 2 | 12 pmol | Twice | Three times |
| 3 | 6 pmol | Twice | Three times |
| 4 | 6 pmol | Twice | Three times |
| 5 | 6 pmol | Once | Three times |

(3) PCR amplification of selection sample

The elution samples obtained in the selection above were subjected to PCR. A PCR reaction solution having the composition shown in Table 5 below was prepared using PrimeSTAR HS DNA Polymerase (manufactured by TAKARA BIO INC.) and was added to the elution samples to perform PCR. The reaction conditions were 98°C for 2 minutes, and then 25 cycles of 98°C for 10 seconds, 62°C for 5 seconds, and 72°C for 35 seconds, followed by reaction at 72°C for 1 minute and then cooling to 10°C. T7omega_New primer having the sequence of SEQ ID NO: 58 and NewYtag for polyA cnvK primer having the sequence of SEQ ID NO: 59 were used (see Table 6). The obtained PCR products were purified using Agencourt AMPure XP in accordance with the manufacturer's instructions.

[Table 5]

| Composition | Capacity (μL) |
|---|---|
| 5× PrimeSTAR Buffer | 10 |
| 2.5 mM dNTP mixture | 4 |
| 10 μM T7omega_New | 1 |
| 10 μM NewYtag for polyA cnvK | 1 |
| Template DNA | 20 |
| 2.5 U/μL PrimeSTAR HS DNA Polymerase | 0.5 |
| Nuclease free water (NFW) | 13.5 |
| Total | 50.0 |

[Table 6]

| Primer | Sequence | SEQ ID NO: |
|---|---|---|
| T7omega_New | 5'-GATCCCGCGAAATTAATACGACTCACTATAGGGGAAGTATTTTTACAACAATTACCAACA-3' | 58 |
| NewYtag for poly A cnvK | 5'-TTTCCACGCCGCCCCCCGTCCT-3' | 59 |

(4) Production of FITC beads

20 μL of streptavidin (SA) magnetic beads (Dynabeads My One Streptavidin C1: Invitrogen) were put in an Eppendorf tube and left to stand on a magnetic plate for 1 minute, and the supernatant was then removed. The beads were re-suspended in 100 μL of 1x binding buffer. After pipetting for 1 minute, the suspension was left to stand on a magnetic plate for 1 minute, and the supernatant was removed. 20 μL of 10 μg/mL Biotin(5-Fluorescein) (Sigma-Aldrich) was added to the resultant, and the mixture was stirred at room temperature for 1 hour and was then left to stand on a magnetic plate for 1 minute, and the supernatant was removed. After addition of 100 μL of PBST, pipetting was performed for 1 minute, followed by leaving to stand on a magnetic plate for 1 minute, and removing the supernatant. This operation was repeated twice, and 1.8 μL of PBST was added to obtain a FITC beads solution.

(5) Sorting by FACS

8 μL of the FITC beads prepared by the procedure above and 4 μL of the SARS-CoV-2 spike S1 protein-immobilized beads were mixed, and the mixture was left to stand on a magnetic plate for 1 minute, and the supernatant was then removed. Re-suspension was performed by addition of 100 μL of PBST. After pipetting for 1 minute and leaving to stand on a magnetic plate for 1 minute, the supernatant was removed to prepare beads for FACS sorting. The obtained beads were mixed with 50 μL of cDNA display solution (6 pmol) prepared from the PCR amplification product in (R5). The mixture was stirred at room temperature for 30 minutes and was then left to stand on a magnetic stand for 2 minutes, and the supernatant was removed. After addition of 100 μL of PBST, pipetting was performed for 1 minute. Subsequently, after leaving to stand on a magnetic plate for 1 minute, the supernatant was removed. This operation was repeated three times, and 1 mL of PBST was added to prepare a solution for sorting.

The obtained solution for sorting was set to FACS (Cell Sorter SH800: SONY Corporation), the sample flow channel and the droplet-forming conditions during sorting were set up, and sorting was performed. The region of magnetic beads was identified from the particle diameter, the regions of the FITC beads and the SARS-CoV-2 spike S1 protein-immobilized beads were identified from the fluorescence intensity by irradiation with 488 nm laser, and sorting was performed under optimum sorting conditions to collect 500,000 particles each with an Eppendorf pipette. Subsequently, centrifugation was performed at 13,000 xg for 10 minutes. After the centrifugation, the resultant was left to stand on a magnetic plate for 10 minutes. Subsequently, the supernatant was removed, and 40 μL of elution buffer was added to the resultant, followed by shaking for 10 minutes. In continuation, after leaving to stand on a magnetic plate for 2 minutes, the eluate was collected. The obtained eluate was purified using Agencourt AMPure XP in accordance with the manufacturer's instructions.

(6) PCR amplification of FACS separation sample

By the same procedure as above, PCR was performed using PrimeSTAR HS DNA Polymerase to obtain a PCR product. The obtained product was purified using Agencourt AMPure XP in accordance with the manufacturer's instructions.

(7) Implementation of amplicon sequence

In order to verify in detail the degree of convergence of the DNA library by in vitro selection cycle, sequence analysis was performed using a next generation sequencer (NGS). Preparation of sequence samples was carried out referring to the method of 2-step PCR Amplicon Library Preparation provided by Illumina, Inc. First, Amplicon PCR was performed using the PCR product after each selection as the template, preparing a PCR reaction solution with the composition shown in Table 7 below, and using primers of SEQ ID NOs: 60 and 61 (see Table 8). The PCR conditions were 98°C for 1 minute and then 15 cycles of 98°C for 10 seconds, 62°C for 5 seconds, and 72°C for 35 seconds, followed by 72°C for 1 minute.

[Table 7]

| Composition | Capacity (μL) |
|---|---|
| 5× PrimeSTAR Buffer | 2 |
| 2.5 mM dNTP mixture | 0.8 |
| 20 μM NGS Fw 1st PCR primer | 0.2 |
| 20 μM NGS Rv 1st PCR primer | 0.2 |
| Template DNA (10 dilution) | 0.5 |
| 2.5 U/μL PrimeSTAR HS DNA Polymerase | 0.1 |
| Nuclease free water (NFW) | 6.2 |
| Total | 10.0 |

[Table 8]

| Primer name | Sequence | SEQ ID NO: |
|---|---|---|
| NGS Fw 1st PCR primer | 5'-TCGTCGGCAGCGTCAGATGTGTATAAGAGACAGNNNNATGGCTGAGGTGCAGCTCGTG-3' | 60 |
| NGS Rv 1st PCR primer | 5'-GTCTCGTGGGCTCGGAGATGTGTATAAGAGACAGNNNNTGATGATGATGGCTACCACCTCCCG-3' | 61 |

The obtained PCR products were purified using Agencourt AMPure XP in accordance with the manufacturer's instructions, and Index PCR was then performed. Index PCR was performed using the PCR products obtained by Amplicon PCR as templates and preparing a PCR reaction solution with the composition shown in Table 9 below. The PCR conditions were 98°C for 1 minute and then 10 cycles of 98°C for 10 seconds, 55°C for 5 seconds, and 72°C for 17 seconds, followed by 72°C for 1 minute.

[Table 9]

| Composition | Capacity (μL) |
|---|---|
| Amplicon 1st PCR product | 2 |
| 5× PrimeSTAR Buffer | 10 |
| 2.5 mM dNTP mixture | 4 |

(continued)

| Composition | Capacity (μL) |
|---|---|
| 10 μM Nextera XT Index 1 Primers (N7XX) (Illumina) | 1 |
| 10 μM Nextera XT Index 2 Primers (S5XX) (Illumina) | 1 |
| 2.5 U/μL PrimeSTAR HS DNA Polymerase | 0.5 |
| Nuclease free water (NFW) | 31.5 |
| Total | 50.0 |

The obtained PCR products were purified using Agencourt AMPure XP in accordance with the manufacturer's instructions, and the sizes of the PCR products were then verified by polyacrylamide gel electrophoresis. Subsequently, the PCR products were each quantitatively measured using Nanodrop, and the molar concentration was calculated in accordance with the following equation (1). Based on the obtained quantitative value, the concentration was adjusted to 4 nM by dilution with NFW.

$$\text{DNA concentration } [ng/\mu L]/(660 \ [g/mol] \times 550 \ [bp]) \times 10^6 = \text{DNA concentration } [nM] \quad (1)$$

A 4 nM library was prepared in accordance with a recommended protocol when being used. The final concentration of the denatured and diluted sample library was adjusted to 7 pM. The PhiX Control in the sample library was adjusted to 5%. Sequencing was performed using MiSeq (Illumina, Inc.) and MiSeq Reagent Nano Kit v2500 cycle (Illumina, Inc.).

(8) Extraction of amino acid sequence of VHH antibody and CDR3 cluster analysis

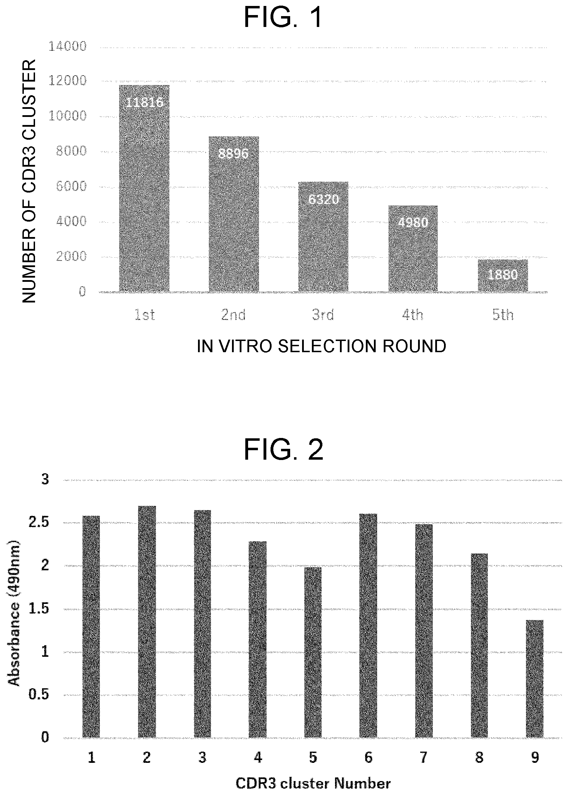

The sequence data demultiplexed using MiSeq Controller were subjected to analysis. Nucleotide sequence regions encoding a VHH antibody was extracted from the sequence data and were translated into amino acid sequences. From the obtained translation data, the number of amino acid sequences that completely agreed with any of the amino acid sequences was counted, and the number of CDR3 clusters was then counted to determine the number of clusters in each round. As a result, the number of clones decreased as rounds progressed, and it was confirmed that good selection could be performed (Figure 1).

(9) Selection of anti-SARS-CoV-2VHH antibody candidate sequences

Clones considered to be non-specific binding were removed by, for example, comparison of the data of FACS sorting, the frequency increase rate (Enrichment) between rounds was then calculated for the CDR3 cluster appeared in R5, and CDR3 clusters in which the appearance frequency increased by more than 10 times from R4 to R5 were extracted. As a result, nine CDR3 clusters shown in Table 10 below corresponded.

[Table 10]

| Cluster No. | CDR3 sequence | Number of CDR3 residues | R5/R4 enrichment | SEQ ID NO: |
|---|---|---|---|---|
| 1 | LLRRYNYGFTFDNY | 14 | 48.54 | 1 |
| 2 | FGGSDFLMDY | 10 | 29.16 | 2 |
| 3 | AWSDYEYLEV | 10 | 20.61 | 3 |
| 4 | TPWYSASHTY | 10 | 46.31 | 4 |
| 5 | VTWLRGDY | 8 | 22.15 | 5 |
| 6 | ITTGSPLLGGGMDF | 14 | 175.16 | 6 |
| 7 | VFPSGGDY | 8 | 30.87 | 7 |
| 8 | VGLFGIQASDY | 11 | 25.12 | 8 |

(continued)

| Cluster No. | CDR3 sequence | Number of CDR3 residues | R5/R4 enrichment | SEQ ID NO: |
|---|---|---|---|---|
| 9 | PGVVTGSYDVRNY | 14 | 26.23 | 9 |

(Example 2) Production of polyclonal VHH antibody by cell-free translation system

(1) Preparation of gene

PCR was performed using the PCR products obtained in R5 of in vitro screening as templates and Newleft primer (SEQ ID NO: 62) and nine specific primers containing the CDR3 sequence selected above. Subsequently, the C-terminal was extended with the Newleft primer and a primer containing a FR4 sequence after CDR3 and a His tag sequence, and finally, PCR was performed using PURE_System_Fw primer (SEQ ID NO: 63) and PURE_System_Rv primer (SEQ ID NO: 64) designed for PUREflex system synthesis (see Table 11 below). The obtained PCR products were purified using Agencourt AMPure XP to prepare nine genes for producing polyclonal VHH antibodies.

[Table 11]

| Primer name | Sequence | SEQ ID NO: |
|---|---|---|
| Newleft | 5'-GATCCCGCGAAATTAATACGACTCACTATAGGG-3' | 62 |
| PURE System_FW | 5'-GAAATTAATACGACTCACTATAGGGAGACCACAACGGTTTCCCTCTAGAAATAATTTTGTTTAACTTTAAGAAGGAGATATACCAATGGCTGAGGTGCAGCTCGTG-3' | 63 |
| PURE_System_Rv | 5'-CCGCCGTGATGATGTTACTTACTTACTTGTCGTCATCGTCTTTGTAGTCGTGATGATGATGATGATGGCTGC-3' | 64 |

(2) Cell-free translation synthesis

Reaction solutions with the composition shown in Table 12 below were prepared using the genes prepared in (1) above as template DNAs in accordance with the protocol of PUREflex 1.0 (GeneFrontier Corporation) and were incubated at 37°C for 3 to 4 hours to synthesize polyclonal VHH antibodies.

[Table 12]

| Composition | Capacity (μL) |
|---|---|
| Solution I | 10 |
| Solution II | 1 |
| Solution III | 1 |
| DNA template (30 ng) | X |
| DNAK mix | 1 |
| Nuclease free water | 7-X |
| Total | 20 |

(3) His tag purification

30 μL of His Mag Sepharose Ni Beads (GE Healthcare) were left to stand on a magnetic plate for 1 minute, and the supernatant was discarded. Subsequently, the beads were re-suspended in 200 μL of a binding buffer. This washing operation was performed twice. 20 μL of the cell-free translation solution prepared in (1) above was added to the tube containing the His Mag Sepharose Ni Beads, and incubation was performed at room temperature for 30 minutes. Subsequently, the tube was left to stand on a magnetic plate for 1 minute, and the supernatant was discarded. 200 μL

of the binding buffer was added to this tube, and pipetting was performed for 1 minute to obtain a suspension. This tube was left to stand on a magnetic plate for 1 minute, and the supernatant was discarded. This washing operation was performed twice. 20 µL of His-tag elution buffer (20 mM sodium phosphate, 500 mM NaCl, 250 mM imidazole, 0.05% Tween 20, pH 7.4) was added to this tube, and incubation was performed at room temperature for 15 minutes. The tube was left to stand on a magnetic plate for 1 minute, and the supernatant was collected. This elution operation was performed twice. A PBS solution containing 1% BSA was added to the tube for diluting to 200 µL to obtain each polyclonal VHH antibody sample solution.

(Example 3) Evaluation by ELISA

SARS-CoV-2 S1 subunit-Fc solution (The Native Antigen Company) was diluted with a PBS solution containing 1% BSA to 2 µg/mL. 100 µL of this solution was added to each well of an immune module (Immuno Clear Standard Modules_C8_MaxiSorp (cat# 445101, Thermo Scientific)). After sealing, the module was left to stand at 4°C overnight to immobilize the S1 protein above. This module was washed with 200 µL of PBST three times, and 300 µL of a PBS solution containing 3% BSA was added to each well, followed by incubation at room temperature for 1 hour. Subsequently, this module was washed with 200 µL of PBST (0.05% Tween 20) three times, and 100 µL of the polyclonal VHH antibody sample solution prepared in (3) of Example 2 above was then added to each well of the S1 protein-immobilized module, followed by shaking at room temperature. Subsequently, this module was washed with 200 µL of PBST three times.

Anti-FLAG-tag mAb conjugated with HRP (cat# A8592, Sigma-Aldrich) was diluted to 1/10,000 with a PBS solution containing 1% BSA. Subsequently, 100 µL of this diluted solution was added to each well, and the well plate was shielded from light and shaken at room temperature for 1 hour. This module was washed with 200 µL of PBST three times. OPD tablet (cat# 155-02161, FUJIFILM Wako Pure Chemical Corporation) was dissolved in 10 mL of 0.1 M NaH$_2$PO$_4$, and 5 µL of 30% H$_2$O$_2$ was added to the solution to prepare an OPD tablet solution. After washing, 100 µL of the OPD tablet solution was added to each well and incubated for 5 minutes in the dark. 100 µL of 1 M sulfuric acid was added to each well, and immediately the absorbance of each well was measured at 490 nm using Microplate Reader Infinite 200Pro M PLEX (TECAN) (Figure 2). The results of this ELISA test demonstrated that all the selected nine polyclonal VHH antibodies had good binding activities.

(Example 4) Production of monoclonal VHH antibody by recombinant *Corynebacterium*

(1) Construction of VHH expression plasmid

VHH-COVE5, VHH-COVE6, VHH-COVE7, VHH-COVE8, and VHH-COVE9 having a linker and a His tag sequence added to the C-terminal were produced by recombinant *Corynebacterium.* Specifically, a VHH antibody (SEQ ID NO: 90) including the same CDR1, CDR2, and CDR3 as those of VHH-COVE5 (SEQ ID NO: 69) (hereinafter, referred to as "*Corynebacterium*-derived VHH-COVE5"), a VHH antibody (SEQ ID NO: 91) including the same CDR1, CDR2, and CDR3 as those of VHH-COVE6 (SEQ ID NO: 70) (hereinafter, referred to as "*Corynebacterium*-derived VHH-COVE6"), a VHH antibody (SEQ ID NO: 92) including the same CDR1, CDR2, and CDR3 as those of VHH-COVE7 (SEQ ID NO: 71) (hereinafter, referred to as "*Corynebacterium*-derived VHH-COVE7"), a VHH antibody (SEQ ID NO: 93) including the same CDR1, CDR2, and CDR3 as those of VHH-COVE8 (SEQ ID NO: 72) (hereinafter, referred to as "*Corynebacterium*-derived VHH-COVE8"), and a VHH antibody (SEQ ID NO: 94) including the same CDR1, CDR2, and CDR3 as those of VHH-COVE9 (SEQ ID NO: 73) (hereinafter, referred to as "*Corynebacterium*-derived VHH-COVE9") were produced. In order to express proteins of the VHH clones including a CDR3 sequence recognized to have the binding by ELISA evaluation, the gene of each VHH was prepared from the DNA library of R5 of in vitro screening and was introduced into an expression plasmid in a form of including a His tag. The synthesized gene of the VHH clone and an empty expression plasmid were treated with two restriction enzymes Sfil and NotI (both available from Thermo Fisher Scientific), and the DNAs of target fragments were each isolated and purified by agarose gel electrophoresis. Subsequently, the VHH clone gene and expression plasmid treated with the restriction enzymes were ligated using Ligation Mix (TOYOBO CO., LTD.), and the ligation product was transformed into competent cell JM109 for cloning. Plasmid was extracted from the transformed *Escherichia coli* to acquire a VHH clone expression plasmid.

(2) Production of recombinant *Corynebacterium glutamicum* strain

The plasmid was introduced into *Corynebacterium glutamicum* strain by an electroporation method described below. Glycerol-stocked *Corynebacterium glutamicum* strain was inoculated in CM2G liquid culture medium and was shake-cultured at 30°C at 160 rpm for several hours. This culture solution was collected in a microtube and was centrifuged with a centrifugal separator (TAITEC Corporation) at 15,000 rpm (20,380 xg) for 1 minute, and the resulting pellet obtained by removing the supernatant was suspended in sterilized water. Again, the bacterial cells were washed with sterilized water by the same operation as above, and the obtained pellet was suspended in 100 $\mu$L of sterilized water to prepare a suspension. This suspension was mixed with various VHH expression plasmids, and electroporation was performed. 1 mL of CM2G culture medium was added to this solution, the mixture was shaken at 30°C at 180 rpm for 1 hour, and an appropriate amount of the mixture was then applied onto a CM2G agar culture medium plate containing 25 $\mu$g/mL kanamycin, followed by incubation at 30°C for 1 to 2 days.

(3) VHH production

The recombinant *Corynebacterium glutamicum* strain produced in (2) above was inoculated in a CM2G culture medium containing 25 $\mu$g/mL kanamycin, followed by shaking at 30°C overnight to obtain a pre-culture solution. The whole amount of the pre-culture solution was inoculated in a PM1S culture medium at 5%, followed by shake-culturing at 25°C for 72 hours. After completion of the culture, the culture supernatant was collected by centrifugation with a centrifugal separator (Thermo Fisher Scientific) at 4,100 rpm at 4°C for 30 minutes. In order to verify the production of VHH in each culture supernatant, SDS-PAGE was performed. 4 $\mu$L of a sample was applied each well, and electrophoresis was then performed under conditions of 150 V for 1 hour. As the molecular weight marker, Precision Plus Protein (TM) Standards (BIO-RAD Laboratories, Inc.) were used. After the electrophoresis, Coomassie brilliant blue (CBB) staining was performed to verify that VHH was synthesized in the culture supernatant.
The collected culture supernatant was filtered through PVDF membrane (Merck Millipore) and then purified using Ni Sepharose 6 Fast Flow (Cytiva) in accordance with the manufacturer's instructions. 400 $\mu$L of His-tag elution buffer (50 mM Tris-HCl, 300 mM NaCl, 500 mM Imidazole, pH 7.5) was added thereto, and centrifugation was performed with a centrifugal separator (TOMY DIGITAL BIOLOGY CO., LTD.) at 500 $\times$g for 30 seconds. The eluate was collected as a VHH antibody sample.

(Example 5) Production of VHH by protease deficient recombinant *Bacillus subtilis*

(1) Artificial synthesis of gene sequence

VHH-COVE1, VHH-COVE2, VHH-COVE3, and VHH-COVE4 having a His tag sequence attached to the C-terminal were produced by using protease deficient recombinant *Bacillus subtilis*. Specifically, artificial synthetic genes of VHH-COVE1 (SEQ ID NO: 95) (hereinafter, referred to as *"Bacillus subtilis*-derived VHH-COVE1"), VHH-COVE2 (SEQ ID NO: 96) (hereinafter, referred to as *"Bacillus subtilis*-derived VHH-COVE2"), VHH-COVE3 (SEQ ID NO: 97) (hereinafter, referred to as *"Bacillus subtilis*-derived VHH-COVE3"), and VHH-COVE4 (SEQ ID NO: 98) (hereinafter, referred to as *"Bacillus subtilis*-derived VHH-COVE4") each having an Ala residue attached to the N-terminal and a His tag sequence including a linker (SEQ ID NO: 74) attached to the C-terminal were synthesized. SEQ ID NO: 77 (VHH-COVE1 expression construct), SEQ ID NO: 78 (VHH-COVE2 expression construct), SEQ ID NO: 79 (VHH-COVE3 expression construct), and SEQ ID NO: 80 (VHH-COVE4 expression construct) in which a termination codon was attached to the 3' end of each of the nucleotide sequences encoding SEQ ID NOs: 95 to 98 and GCAGCTCTTGCAGCA (SEQ ID NO: 75) and TCTATTAAACTAGTT (SEQ ID NO: 76) were further attached to the 5' end and the 3' end, respectively, were artificially synthesized by Eurofins Genomics K.K. They were used for experiments.

(2) Construction of VHH expression plasmid with His tag

The plasmid sequence for constructing expression plasmid was amplified by performing PCR using the recombinant plasmid pHY-S237 (see JP-A-2014-158430) produced based on pHY300PLK as a template and using 5'-GATCCCCGGGAATTCCTGTTATAAAAAAAGG-3' (SEQ ID NO: 81) and 5'-ATGATGTTAAGAAAGAAAACAAAGCAG-3' (SEQ ID NO: 82) as primers and PrimeSTAR Max DNA Polymerase (TAKARA BIO INC.).
PCR was performed using genomes of 168 strains as templates and a primer set of 5'-GAATTCCCGGGGATCTAAGAAAAGTGATTCTGGGAGAG-3' (SEQ ID NO: 83) and 5'-CTTTCTTAACATCATAGTAGTTCACCACCTTTTCCC-3' (SEQ ID NO: 84) to amplify a promoter DNA derived from spoVG gene.
The obtained promoter DNA was incorporated into the plasmid sequence using In-Fusion HD Cloning Kit (TAKARA BIO INC.) to construct VHH expression plasmid linked to spoVG promoter.
The plasmid sequence above was amplified by performing PCR using 5'-TGCTGCAAGAGCTGCCGGAAATAAA-3' (SEQ ID NO: 85) and 5'-TCTATTAAACTAGTTATAGGG-3' (SEQ ID NO: 86) as primers and PrimeSTAR Max DNA Polymerase (TAKARA BIO INC.). The DNAs including the artificially synthesized genes produced in (1) were each incorporated into the obtained PCR products by using In-Fusion HD Cloning Kit (TAKARA BIO INC.) to construct VHH expression plasmids with His tag that includes respective artificially synthesized VHH genes. In accordance with the procedure shown in (3) below, the constructed plasmids were introduced into strains (Dpr8ΔsigF) obtained from a *Bacillus subtilis* 168 strain by causing deficiencies of eight extracellular protease genes (epr, wprA, mpr, nprB, bpr, nprE, vpr, aprE) in accordance with the method described in JP-A-2006-174707 and further causing a deficiency of sigF gene involving in sporulation in accordance with the method described in JP-B-4336082.

(3) Production of recombinant *Bacillus subtilis*

The introduction of the plasmid into a *Bacillus subtilis* strain was performed by a protoplast method shown below. *Bacillus subtilis* stored in a solution containing glycerol was inoculated in a tube containing 1 mL of LB liquid culture medium and was shake-cultured at 30°C at 210 rpm overnight. On the following day, 10 $\mu$L of the culture solution in this tube was inoculated in 1 mL of fresh LB liquid culture medium and was shake-cultured at 37°C at 210 rpm for about 2 hours. After completion of the culture, this culture solution was collected in a 15-mL tube and was centrifuged at 12,000 rpm for 5 minutes, and the supernatant was removed. The resulting pellet was suspended by adding 500 $\mu$L of SMMP containing 4 mg/mL lysozyme (Sigma-Aldrich) to the tube, and this tube was subjected to incubation at 37°C for 1 hour.

After completion of the incubation, this tube was centrifuged at 3,500 rpm for 10 minutes, and the supernatant was removed. The resulting pellet was suspended by adding 400 $\mu$L of SMMP to the tube to obtain a suspension. 33 $\mu$L of the obtained suspension was added to each of other tubes, each plasmid produced as described above was added thereto and mixed, and 100 $\mu$L of 40% PEG was further added thereto, followed by vortex to obtain each mixture solution. This mixture solution was inversion-mixed with 350 $\mu$L of SMMP, followed by shaking at 30°C at 210 rpm for 1 hour to obtain a shake culture product. Subsequently, the whole amount of the shake culture product was applied onto an agar culture medium plate containing DM3 agar culture medium prepared in advance and was incubated at 30°C for 2 to 3 days to obtain recombinant *Bacillus subtilis.*

(4) Production of VHH

The recombinant *Bacillus subtilis* produced in (3) was inoculated in 1 mL of LB culture medium containing 50 ppm tetracycline, followed by reciprocal shaking at 32°C overnight to obtain a pre-culture solution. The obtained pre-culture solution was inoculated in a 20 mL of 2×L-mal culture medium at 1% in a baffled conical flask and was shake-cultured at 30°C for 72 hours. At the time of completion of the culture, 1 mL of the culture product was put in a microtube and was centrifuged at 4°C at 15,000 rpm for 5 minutes, and the supernatant was collected. Each VHH contained in the collected supernatant was purified using Ni-NTA Agarose Beads (FUJIFILM Wako Pure Chemical Corporation) in accordance with the protocol attached to the kit. As the eluate in the purification, PBS containing 50 mM imidazole was used.

In order to verify the amount of the produced antibodies contained in the culture supernatant, Western blotting was performed under the following conditions. SDS-PAGE used was SuperSep Ace or 15-20% (Tricine Gel) (both available from FUJIFILM Wako Pure Chemical Corporation). A sample containing 0.5 $\mu$L of the culture supernatant was applied to each well and then subjected to electrophoresis at 120 V for 3 hours. As the molecular weight marker, xL ladder (Broad) (APRO Science) was used. The protein was transferred from the SDS-PAGE gel to PVDF membrane using Trans-Blot Turbo Mini PVDF Transfer Packs (BIO-RAD Laboratories, Inc.) and Trans-Blot Turbo System (BIO-RAD Laboratories, Inc.). Antibody response was performed by iBind Western System (Invitrogen) using 6x-His Tag monoclonal antibody (3D5) as the antibody and HRP (Invitrogen) or ANTI-FLAG M2-peroxidase (HRP) conjugate (Sigma-Aldrich). A target protein was detected using 1-Step Ultra TMB-Blotting Solution (Thermo Scientific).

(Example 6) Measurement of binding activity by bio-layer interferometry

Binding activities of *Bacillus subtilis*-derived VHH-COVE1 to VHH-COVE4 and *Corynebacterium*-derived VHH-COVE5 to VHH-COVE9 to SARS-CoV-2 (2019-nCoV) spike S1 protein were measured using bio-layer interferometry (hereinafter, may be abbreviated to "BLI"). In the BLI, each VHH clone adjusted to 10 to 20 $\mu$g/mL with a kinetic buffer (0.05% Tween 20-containing PBS, pH 7.4) was immobilized to an Anti-Penta-HIS (HIS1K, ForteBio) sensor chip using Octet 384 (ForteBio) and was bound to SARS-CoV-2 (2019-nCoV) spike Sl-Fc recombinant protein (Sino Biological, Inc.) prepared by 2-fold serial dilution starting at 495 nM, and the dissociation rate was measured. As preparation for measurement, in order to hydrate the sensor chip, the tip of the sensor chip was immersed in 200 $\mu$L of a kinetic buffer for 10 minutes. Subsequently, 50 $\mu$L of each measurement solution was added to a 384-well black plate (ForteBio), and measurement was carried out by the steps 1) to 6) shown below.

    1) Baseline step: baseline measurement in kinetic buffer (60 seconds);
    2) Loading step: immobilization of VHH antibody to sensor (240 seconds);
    3) Baseline step: baseline measurement in kinetic buffer (30 seconds);
    4) Association step: association with spike S1 protein solution (180 seconds);
    5) Dissociation step: measurement in kinetic buffer (240 seconds); and

6) Regeneration step: repeating measurement in glycine-HCl (pH 2.2) for 5 seconds and measurement in kinetic buffer for 5 seconds three times.

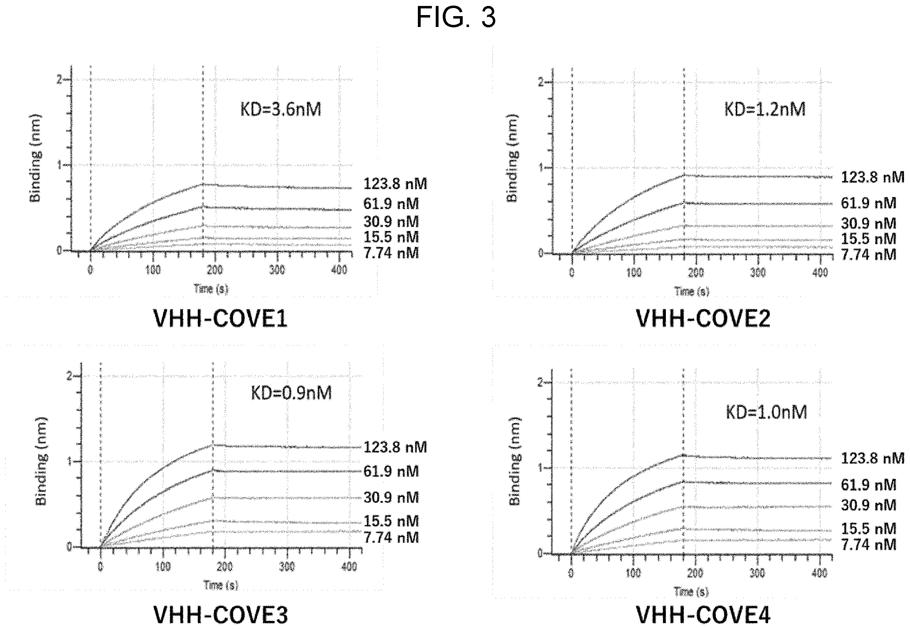

After completion of the measurement above, the reference (kinetic buffer alone) was subtracted from the actual measurement values, global fitting was performed using Octet software and 1 : 1 binding model, and the binding activity was calculated (Figure 3). The equilibrium dissociation constant (KD) of each VHH clone with respect to spike S1 protein is shown in Table 13.

[Table 13]

| Antibody | $K_D$ (M) | $K_{on}$ (Ms$^{-1}$) | $K_{off}$ (s$^{-1}$) | SEQ ID NO: |
|---|---|---|---|---|
| VHH-COVE1 | $3.63 \times 10^{-9}$ | $7.70 \times 10^4$ | $2.80 \times 10^{-4}$ | 1 |
| VHH-COVE2 | $1.20 \times 10^{-9}$ | $7.07 \times 10^4$ | $8.47 \times 10^{-5}$ | 2 |
| VHH-COVE3 | $9.24 \times 10^{-10}$ | $1.06 \times 10^5$ | $9.75 \times 10^{-5}$ | 3 |
| VHH-COVE4 | $9.97 \times 10^{-10}$ | $1.15 \times 10^5$ | $1.14 \times 10^{-4}$ | 4 |
| VHH-COVE5 | $1.30 \times 10^{-12}$ | $3.72 \times 10^4$ | $< 1.0 \times 10^{-7}$ | 5 |
| VHH-COVE6 | $9.09 \times 10^{-10}$ | $8.04 \times 10^4$ | $7.31 \times 10^{-5}$ | 6 |
| VHH-COVE7 | $<1.0 \times 10^{-12}$ | $6.96 \times 10^4$ | $< 1.0 \times 10^{-7}$ | 7 |
| VHH-COVE8 | $2.37 \times 10^{-9}$ | $2.33 \times 10^4$ | $5.51 \times 10^{-5}$ | 8 |
| VHH-COVE9 | $7.26 \times 10^{-10}$ | $1.92 \times 10^5$ | $1.39 \times 10^{-4}$ | 9 |

(Example 7) Measurement of competitive inhibition activity by bio-layer interferometry

Competitive inhibition between VHH clones with respect to SARS-CoV-2 spike S1 protein was analyzed using Octet 384. The competitive inhibition activity was calculated by comparing a binding signal obtained by immersing a sensor chip to which any of *Bacillus subtilis*-derived VHH-COVE1 to VHH-COVE4 and *Corynebacterium*-derived VHH-COVE5 to VHH-COVE9 was immobilized as a first antibody in a mixture solution of spike S1 protein adjusted to a certain concentration and any of *Bacillus subtilis*-derived VHH-COVE1 to VHH-COVE4 and *Corynebacterium*-derived VHH-COVE5 to VHH-COVE9 as a second antibody for a certain time and a binding signal when only spike S1 protein was bound.
First, a first competitive VHH antibody adjusted to 100 μg/mL with a kinetic buffer (0.05% Tween 20-containing PBS, pH 7.4) was immobilized to a HIS1K sensor chip. Secondly, 100 μg/mL of a second competitive VHH clone was mixed with 20 μg/mL, 10 μg/mL, 5 μg/mL, or 0 μg/mL SARS-CoV-2 (2019-nCoV) spike SI-Fc recombinant protein (Sino Biological, Inc.), followed by reacting for 5 minutes to prepare a binding solution. The order of measurement in each run was as follows.

1) Baseline step: baseline measurement in kinetic buffer (60 seconds);
2) Loading step: immobilization of VHH antibody to sensor (60 seconds);
3) Baseline step: baseline measurement in kinetic buffer (30 seconds);
4) Association step: association with spike S1 protein solution in competitive VHH antibody mixture solution (120 seconds);
5) Dissociation step: measurement in kinetic buffer (30 seconds); and
6) Regeneration step: repeating measurement in glycine-HCl (pH 2.2) for 5 seconds and measurement in kinetic buffer for 5 seconds three times.

Regarding the competitive inhibition rate, the binding signal obtained when the first antibody and spike S1 protein were used was defined as 100%, and when the signal intensity in the presence of the second antibody was reduced to less than 25% of the maximum binding capacity, it was defined as competition, and when the binding exceeded 75%, it was defined as non-competition. The level between 25% and 75% was defined as mean competition. It was inferred from the results of binding competition signal of each clone that VHH clones having high binding activity are obtained and that VHH clones recognized to have activity this time can be divided into at least five competition groups (see Table 14 below).

[Table 14]

| Competition group | | A | B | C | A | D | C | B | C | E |
|---|---|---|---|---|---|---|---|---|---|---|
| | | COVE1 | COVE2 | COVE3 | COVE4 | COVE5 | COVE6 | COVE7 | COVE8 | COVE9 |
| A | COVE1 | 22.02 | 73.33 | 96.34 | 10.22 | 61.16 | 99.20 | 69.01 | 107.16 | 106.85 |
| B | COVE2 | 62.71 | 18.62 | 17.02 | 68.40 | 33.86 | 3.38 | 5.54 | 3.61 | 2.13 |
| C | COVE3 | 108.78 | 19.37 | 13.24 | 80.26 | 49.63 | 15.26 | 21.89 | 20.09 | 5.87 |

(Example 8) Measurement of neutralizing activity against SARS-CoV-2

The neutralizing activity of *Bacillus subtilis*-derived VHH-COVE2 against SARS-CoV-2 was measured. The antibody was adjusted to a concentration of 15 μg/mL by mixing with 2% Fetal Bovine Serum (FBS)/Dulbecco Modified Eagle Medium (DMEM). Furthermore, a 3-fold diluted solution was prepared by mixing 100 μL of VHH clone mixture solution and 100 μL of 2% FBS/DMEM to prepare 3-fold dilution series solution of the antibody.

100 μL of the 3-fold dilution series solution of the VHH clone was added to a 96-well plate, and 100μL of 2.5 × 10⁶ RNA copies/mL of SARS-CoV-2 solution (obtained from the National Institute of Infectious Diseases) was further added. Subsequently, incubation was performed at 37°C for 2 hours and then at 4°C overnight to contact the antibody with the virus. 200 μL of the solution in which the antibody and the virus were in contact was added to the wells in which vero-E6/TMPRSS2 cells (purchased from JCRB Cell Bank) were inoculated, followed by culturing at 37°C in a 5% $CO_2$ environment. The culture supernatant was collected 3 days after the start of the culturing, and the number of viral genome copies in the supernatant was counted by quantitative PCR.

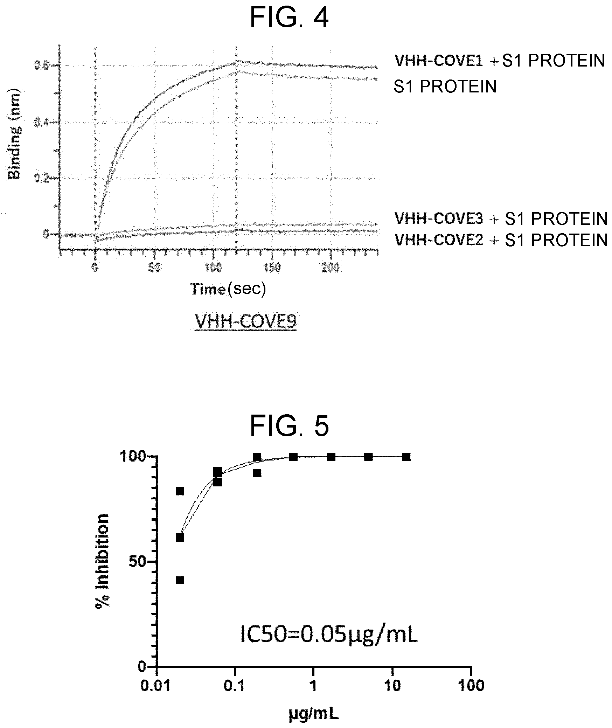

Based on the results obtained by quantitative PCR, the infection inhibition rate to SARS-CoV-2 at each concentration of the VHH clone was calculated (Figure 5). As a result, it was demonstrated that VHH-COVE2 showed neutralizing activity against SARS-CoV-2 and therefore can also bind to particles of SARS-CoV-2. In addition, it was demonstrated that the IC50 determined from the infectins inhibition rate was 0.05 μg/mL and that VHH-COVE2 has high neutralizing activity against SARS-CoV-2.

(Example 9) Production of VHH by protease deficient recombinant *Bacillus subtilis*

VHH-COVE5, VHH-COVE8, and VHH-COVE9 each having a His tag sequence attached to the C-terminal were produced by protease deficient recombinant *Bacillus subtilis.* Specifically, artificial synthetic genes of VHH-COVE5 (SEQ ID NO: 99) (hereinafter, referred to as *"Bacillus* subtilis-derived VHH-COVE5"), VHH-COVE8 (SEQ ID NO: 100) (hereinafter, referred to as *"Bacillus subtilis*-derived VHH-COVE8"), and VHH-COVE9 (SEQ ID NO: 101) (hereinafter, referred to as *"Bacillus subtilis*-derived VHH-COVE9") each having an Ala residue attached to the N-terminal and a His tag sequence including a linker (SEQ ID NO: 74) attached to the C-terminal were synthesized. SEQ ID NO: 87 (VHH-COVE5 expression construct), SEQ ID NO: 88 (VHH-COVE8 expression construct), and SEQ ID NO: 89 (VHH-COVE9 expression construct) in which a termination codon was attached to the 3' end of each of the nucleotide sequences encoding SEQ ID NOs: 99 to 101 and GCAGCTCTTGCAGCA (SEQ ID NO: 75) and TCTATTAAACTAGTT (SEQ ID NO: 76) were further attached to the 5' end and the 3' end, respectively, were artificially synthesized by Eurofins Genomics K.K. They were used for experiments. The subsequent operation for producing VHH by protease deficient recombinant *Bacillus subtilis* was performed in accordance with Example 5.

(Example 10) Measurement of neutralizing activity against SARS-CoV-2

The neutralizing activity against SARS-CoV-2 was evaluated in accordance with the method described in Example 8 above by changing the VHH clone used from VHH-COVE2 to *Bacillus subtilis*-derived VHH-COVE5, *Bacillus subtilis*-derived VHH-COVE8, and *Bacillus subtilis*-derived VHH-COVE9. As a result, the IC50 determined from the infection inhibition rates were 3.048 μg/mL (VHH-COVE5), less than 1.733 μg/mL (VHH-COVE8), and less than 0.58 μg/mL (VHH-COVE9).

(Example 11) Production of VHH by protease deficient recombinant *Bacillus subtilis*

VHH-COVE6 and VHH-COVE7 each having a His tag sequence attached to the C-terminal were produced by protease deficient recombinant *Bacillus subtilis.* Specifically, artificial synthetic genes of VHH-COVE6 (SEQ ID NO: 102) (hereinafter, referred to as *"Bacillus subtilis*-derived VHH-COVE6") and VHH-COVE7 (SEQ ID NO: 103) (hereinafter, referred to as *"Bacillus subtilis*-derived VHH-COVE7") each having an Ala residue attached to the N-terminal and a His tag sequence including a linker (SEQ ID NO: 74) attached to the C-terminal were synthesized. SEQ ID NO: 104 (VHH-COVE6 expression construct) and SEQ ID NO: 105 (VHH-COVE7 expression construct) in which a termination codon was attached to the 3' end of the nucleotide sequences encoding SEQ ID NO: Q and SEQ ID NO: R and GCAGCTCTTGCAGCA (SEQ ID NO: 75) and TCTATTAAACTAGTT (SEQ ID NO: 76) were further attached to the 5' end and the 3' end, respectively, were artificially synthesized by Eurofins Genomics K.K. They were used in experiments. The subsequent operation for producing VHH by protease deficient recombinant *Bacillus subtilis* was performed in accordance with Example 5.

(Example 12) Measurement of binding activity to SARS-CoV-2 variant strain

The binding activities of eight VHH antibodies, *Bacillus subtilis*-derived VHH-COVE1 to VHH-COVE5 and VHH-COVE7 to VHH-COVE9 to the spike protein of the SARS-CoV-2 variant strain were measured by ELISA. As the target proteins, Trimeric SARS-CoV-2 spike antigen full-length (referred to as "Wuhan type"), SARS-CoV-2 full-length Spike Recombinant Antigen B.1.1.7 Mutation (referred to as "British type"), SARS-CoV-2 full-length Spike Recombinant Antigen B.1.351 Mutation (referred to as "South Africa type"), and SARS-CoV-2 full-length Spike Recombinant Antigen P.1 Mutation (referred to as "Brazil type") (Bio-Serv) each diluted with PBST (Phosphate Buffered Saline with 0.05% Tween 20) to 0, 5, 50, and 500 ng/mL were used. The British type is provided by making, to the Wuhan type, H69-V70 deletion, Y144deletion, and N501Y, A570D, D614G, P681H, T716I, S982A, and D1118H mutations. The South Africa type is provided by making, to the Wuhan type, Y144 deletion and K417N, E484K, N501Y, A570D, D614G, P681H, T716I, S982A, and D1118H mutations. The Brazil type is provided by making, to the Wuhan type, L18F, T20N, P26S, D138Y, R190S, K417T, E484K, N501Y, H655Y, T1027I, and V1176F mutations.

100 µL of each VHH antibody adjusted to 20 µg/mL was added to each well of Pierce (TM) Nickle Coated Plates, Clear, 96-well (Thermo Fisher Scientific) and was incubated at room temperature for 1 hour to perform immobilization. Subsequently, the VHH antibody was carefully removed using a pipette, and 200 µL of PBST was then added thereto and carefully removed with a pipette. This operation (referred to as "washing") was repeated three times. Subsequently, 200 µL of 5% skim milk/PBST was added to each well, followed by incubation at room temperature for 1 hour to perform blocking. Subsequently, the skim milk/PBST was carefully removed using a pipette, and the washing operation was then repeated three times. Subsequently, 100 µL of a target protein adjusted to an appropriate concentration was added to each cell, followed by incubation at room temperature for 1 hour. Subsequently, the target protein solution was carefully removed using a pipette, and the washing operation was then repeated three times.

A primary antibody was prepared by diluting Ms mAb to Rhodopsin [1D4] (Abcam) to 1/5,000 with PBST, and 100 µL thereof was added to each well. After incubation at room temperature for 1 hour, the primary antibody was carefully removed using a pipette. The washing operation was then repeated three times. A secondary antibody was prepared by diluting Goat pAb to Ms (HRP) (Abcam) to 1/5,000 with PBST, and 100 µL thereof was added to each well. After incubation under room temperature and dark conditions for 1 hour, the secondary antibody was carefully removed with a pipette, and the washing operation was then repeated three times. A luminescent substrate was prepared by dissolving OPD tablet (Thermo Fisher Scientific) in Stable Peroxide Substrate buffer (Thermo Fisher Scientific), and 100 µL thereof was added to each well. Subsequently, incubation was performed under room temperature and dark conditions for 20 minutes, and the absorbance (450 nm) was measured using GloMax (R) Explorer System (Promega Corporation).

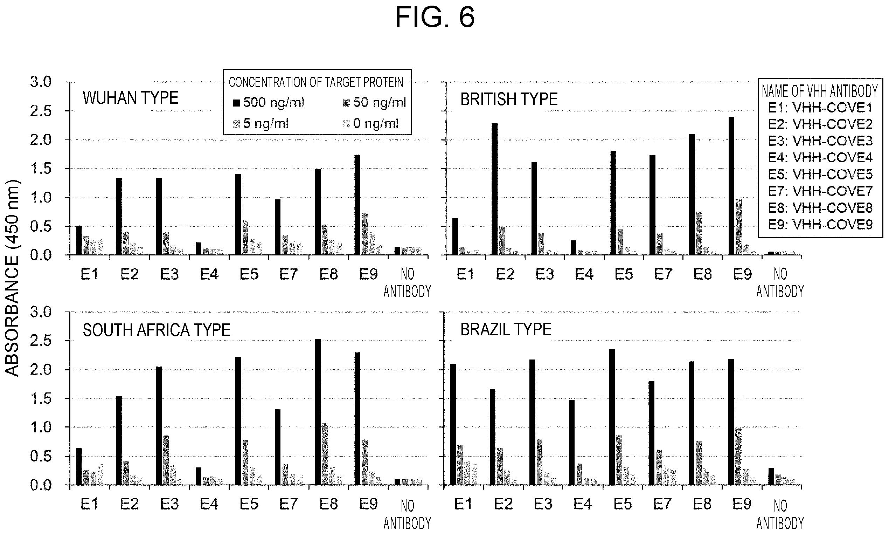

The results of ELISA test are shown in Figure 6. In all VHH antibodies used in the test, binding activity to four spike proteins was observed. In VHH antibodies excluding E1 and E4, particularly good binding activity (absorbance of 0.97 or more) to every spike protein was observed.

(Example 13) Measurement of binding activity to SARS-CoV-2 variant strain

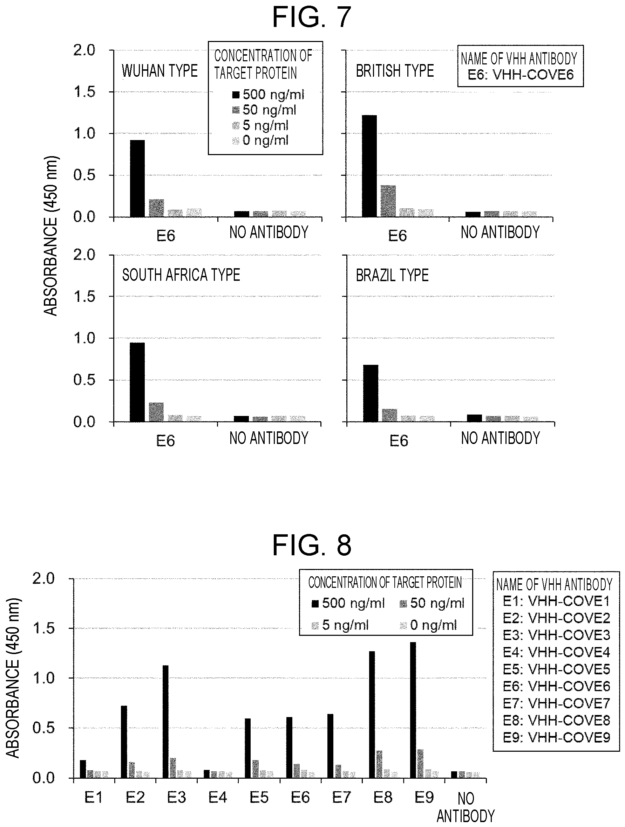

In accordance with the method described in Example 11 above, the binding activity of *Bacillus subtilis*-derived VHH-COVE6 to spike proteins of SARS-CoV-2 variant strains was measured by ELISA. As in Example 11, as the target proteins, Trimeric SARS-CoV-2 spike antigen, full-length (referred to as "Wuhan type"), SARS-CoV-2 full-length Spike Recombinant Antigen B.1.1.7 Mutation (referred to as "British type"), SARS-CoV-2 full-length Spike Recombinant Antigen B.1.351 Mutation (referred to as "South Africa type"), and SARS-CoV-2 full-length Spike Recombinant Antigen P.1 Mutation (referred to as "Brazil type") (Bio-Serv) each diluted with PBST (Phosphate Buffered Saline with 0.05% Tween 20) (to 0, 5, 50, and 500 ng/mL) were used. The mutation sites in each variant strain were the same as those in Example 12. The results of the ELISA test are shown in Figure 7, and clear binding activity of VHH-COVE6 was observed also to the spike protein of every variant strain.

(Example 14) Measurement of binding activity to SARS-CoV-2 variant strain

In accordance with the method described in Example 11 above, the binding activities of nine VHH antibodies, *Bacillus subtilis*-derived VHH-COVE1 to VHH-COVE9, to spike protein of SARS-CoV-2 British/Nigeria variant strain were measured by ELISA. As the target protein, SARS-CoV-2 full-length Spike Recombinant Antigen B.1.525 Mutation (hereinafter, referred to as "British/Nigeria type") (Bio-Serv) diluted with PBST (Phosphate Buffered Saline with 0.05% Tween 20) (to 0, 5, 50, and 500 ng/mL) was used. The British/Nigeria variant strain is provided by making, to the Wuhan type, H69-V70deletion, Y144deletion, and Q52R, E484K, Q667H, and F888L mutations.

The results of the ELISA test are shown in Figure 8. In all VHH antibodies used in the test, binding activity to spike protein of British/Nigeria type was observed. In VHH-COVE3, VHH-COVE8, and VHH-COVE9, very good binding activity (absorbance of 1 or more) was observed. In also VHH-COVE5, VHH-COVE6, and VHH-COVE7, good binding activity (absorbance of 0.5 or more) was observed.

(Example 15) Measurement of binding activity to SARS-CoV-2 variant strain

In accordance with the method described in Example 11 above, the binding activities of nine VHH antibodies, *Bacillus subtilis*-derived VHH-COVE1 to VHH-COVE9, to spike proteins of SARS-CoV-2 California variant strain and India variant strain were measured by ELISA. As the target proteins, SARS-CoV-2 full-length Spike Recombinant Antigen B.1.429 Mutation (hereinafter, referred to as "California type") and SARS-CoV-2 full-length Spike Recombinant Antigen B.1.617 Mutation (hereinafter, referred to as "India type) (Bio-Serv) each diluted with PBST (Phosphate Buffered Saline with 0.05% Tween 20) (to 0, 5, 50, and 500 ng/mL) were used. The California variant strain is provided by making, to the Wuhan type, S13I, W152C, L452R, and D1183Y mutations. The India mutant strain is provided by making, to the Wuhan type, G142D, E154K, L452R, E484Q, D614G, P681R, and Q1071H mutations.

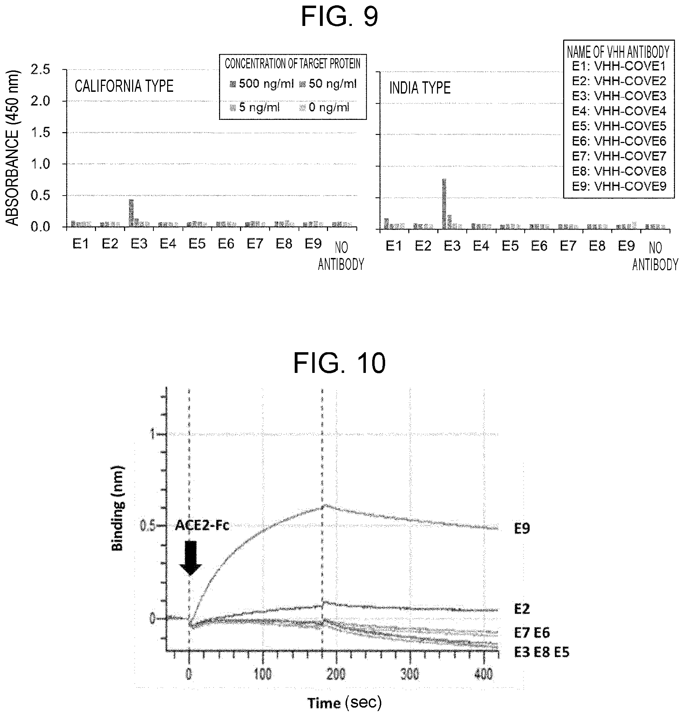

The results of the ELISA test are shown in Figure 9. In all VHH antibodies excluding VHH-COVE3, no binding activity to spike proteins of California type and India type was observed. In contrast, in VHH-COVE3, high binding activities to spike proteins of California type and India type were observed. Based on also Example 11 and Example 13, it was revealed that VHH-COVE3 recognizes an epitope that is hardly influenced by mutation of spike protein and exhibits broad binding activity to variant strains of SARS-CoV-2.

(Example 16) Measurement of binding activity to SARS-CoV-2 spike RBD

The binding activities of nine VHH antibodies, *Bacillus subtilis*-derived VHH-COVE1 to VHH-COVE9, to SARS-CoV-2 spike RBD recombinant protein were measured by the same method as in Example 7. Each VHH clone adjusted to 10 to 20 $\mu$g/mL with a kinetic buffer (PBS containing 0.05% Tween 20, pH 7.4) was immobilized on an Anti-Penta-HIS (HIS1K, ForteBio) sensor chip and was allowed to bind to SARS-CoV-2 spike RBD recombinant protein (40592-VNAH, Sino Biological, Inc., Wuhan type) prepared by 2-fold serial dilution starting at 199.2 nM, and the dissociation rate was measured. As preparation for measurement, in order to hydrate the sensor chip, the tip of the sensor chip was immersed in 200 $\mu$L of the kinetic buffer for 10 minutes. Subsequently, 50 $\mu$L of each measurement solution was added to a 384-well black plate (ForteBio), and measurement was carried out by the steps 1) to 6) shown below.

1) Baseline step: baseline measurement in kinetic buffer (30 seconds);
2) Loading step: immobilization of VHH antibody to sensor (120 seconds);
3) Baseline step: baseline measurement in kinetic buffer (60 seconds);
4) Association step: association with RBD protein solution (180 seconds);
5) Dissociation step: measurement in kinetic buffer (240 seconds); and
6) Regeneration step: repeating measurement in glycine-HCl (pH 2.2) for 5 seconds and measurement in kinetic buffer for 5 seconds three times.

After completion of the measurement above, the reference (kinetic buffer alone) was subtracted from the actual measurement values, global fitting was performed using Octet software and 1 : 1 binding model, and the binding activity was calculated. The equilibrium dissociation constant ($K_D$) of each VHH antibody with respect to RBD protein is shown in Table 15.

[Table 15]

| Antibody | $K_D$ (M) | $K_{on}$ (Ms$^{-1}$) | $K_{off}$ (s$^{-1}$) | SEQ ID NO: |
|---|---|---|---|---|
| VHH-COVE1 | ND | ND | ND | 1 |
| VHH-COVE2 | $6.42 \times 10^{-9}$ | $3.22 \times 10^4$ | $2.06 \times 10^{-4}$ | 2 |
| VHH-COVE3 | $6.19 \times 10^{-9}$ | $4.09 \times 10^4$ | $2.53 \times 10^{-4}$ | 3 |
| VHH-COVE4 | ND | ND | ND | 4 |
| VHH-COVE5 | $5.40 \times 10^{-8}$ | $1.66 \times 10^4$ | $8.99 \times 10^{-4}$ | 5 |
| VHH-COVE6 | $5.98 \times 10^{-9}$ | $7.89 \times 10^4$ | $4.72 \times 10^{-4}$ | 6 |
| VHH-COVE7 | $4.54 \times 10^{-10}$ | $6.88 \times 10^4$ | $3.13 \times 10^{-5}$ | 7 |
| VHH-COVE8 | $3.19 \times 10^{-8}$ | $2.12 \times 10^4$ | $6.77 \times 10^{-4}$ | 8 |
| VHH-COVE9 | $6.14 \times 10^{-9}$ | $1.69 \times 10^5$ | $1.04 \times 10^{-3}$ | 9 |

From the equilibrium dissociation constant ($K_D$) against RBD, VHH antibodies excluding VHH-COVE1 and VHH-COVE4 showed strong binding activities like those to S1 protein. In contrast, VHH-COVE1 and VHH-COVE4 did not show clear binding. Since it was observed that VHH-COVE1 and VHH-COVE4 bind to S1 protein and compete with VHH-COVE2 and so on for binding to RBD, it was suggested that VHH-COVE1 and VHH-COVE4 recognize a c as an epitope.

(Example 17) Measurement of competitive inhibition activity to binding of SARS-CoV-2 spike RBD and ACE2

Seven VHH antibodies, *Bacillus subtilis*-derived VHH-COVE2, *Bacillus subtilis*-derived VHH-COVE3, and *Bacillus subtilis*-derived VHH-COVE5 to VHH-COVE9, that were recognized to bind to RBD in Example 15 were evaluated for competitive inhibition to the binding of SARS-CoV-2 spike RBD to human ACE2 by the same method as in Example 7 using Octet 384. Each VHH clone adjusted to 10 to 20 $\mu$g/mL with a kinetic buffer (PBS containing 0.05% Tween 20, pH 7.4) was immobilized on an Anti-Penta-HIS (HIS1K, ForteBio) sensor chip by incubation for 120 seconds and was immersed in 199.2 nM SARS-CoV-2 spike RBD recombinant protein as a 1st analyte (20 $\mu$g/mL, 40592-VNAH, Sino Biological, Inc., Wuhan type) solution. The binding after 180 seconds was verified, and the sensor chip was then immersed in the buffer solution for 240 seconds. The sensor chip was immersed in a well of ACE2-Fc adjusted to 20 ng/mL before and after the baseline step of 30 seconds, and the presence or absence of the binding after 180 seconds was verified.
After confirmation of binding response between each VHH antibody immobilized on the sensor chip and RBD as the 1st analyte, ACE2-Fc as a 2nd analyte was allowed to bind thereto. As a result, VHH-COVE3, VHH-COVE5, VHH-COVE6, VHH-COVE7, and VHH-COVE8 clearly inhibited the binding with ACE2 to show competitivity. This result suggests that VHH-COVE3, VHH-COVE5, VHH-COVE6, VHH-COVE7, and VHH-COVE8 show neutralizing activity to SARS-CoV-2.
In contrast, VHH-COVE9 did not show competitivity with ACE2, and VHH-COVE2 showed partial competitivity with ACE2. The change in each sensorgram to ACE2 is shown in Figure 10. This result shows that the epitope of VHH-COVE9 is other than the ACE2-binding site on RBD, and it was inferred that the neutralizing activity thereof is an action that is different from that of general antibodies showing neutralizing activity by direct competition with ACE2.

(Example 18) Measurement of binding activity to SARS-CoV2 spike RBD variant strain

The binding activities of six VHH antibodies, *Bacillus subtilis*-derived VHH-COVE2, *Bacillus subtilis*-derived VHH-COVE3, and *Bacillus subtilis*-derived VHH-COVE5 to VHH-COVE9, to SARS-CoV-2 spike RBD variant strain were measured by the same method as in Example 7. Each of SARS-CoV-2 spike RBD variant strain (SPD-C52Hn, Aero Biosystems, N501Y), SARS-CoV2 spike RBD variant strain (SRD-C52H3, Aero Biosystems, E484K), SARS-CoV2 spike RBD variant (SRD-C52H2, Aero Biosystems, N440K), and SARS-CoV2 spike RBD variant strain (SPD-C52Hp, Aero Biosystems, K417N, E484K, N501Y) was biotinylated using EZ-Link NHS-PEG12-biotinylation reagent (Thermo Fisher Scientific) in advance and was adjusted to 10 to 20 $\mu$g/mL with a kinetic buffer (PBS containing 0.05% Tween 20, pH 7.4). RBD variant strain was immobilized on an SA sensor chip (ForteBio) and was allowed to bind to each VHH prepared by 2-fold serial dilution starting at 666.7 nM, and the dissociation rate was measured. As preparation for measurement, in order to hydrate the sensor chip, the tip of the sensor chip was immersed in 200 $\mu$L of the kinetic buffer for 10 minutes. Subsequently, 50 $\mu$L of each measurement solution was added to a 384-well black plate (ForteBio), and measurement was carried out by the steps 1) to 5) shown below.

1) Baseline step: baseline measurement in kinetic buffer (60 seconds);
2) Loading step: immobilization of VHH antibody to sensor (300 seconds);
3) Baseline step: baseline measurement in kinetic buffer (60 seconds);
4) Association step: association with spike RBD protein solution (120 seconds); and
5) Dissociation step: measurement in kinetic buffer (240 seconds).

After completion of the measurement above, the reference (kinetic buffer alone) was subtracted from the actual measurement values, global fitting was performed using Octet software and 1 : 1 binding model, and the binding activity was calculated. The equilibrium dissociation constant ($K_D$) of each VHH clone with respect to SARS-CoV2 spike RBD variant strain is shown in Tables 16, 17, 18, and 19.

[Table 16]

| Binding affinity to RBD variant strain (N501Y) | | | | |
|---|---|---|---|---|
| Antibody | $K_D$ (M) | $K_{on}$ (Ms$^{-1}$) | $K_{off}$ (s$^{-1}$) | SEQ ID NO: |
| VHH-COVE2 | $4.01 \times 10^{-9}$ | $1.09 \times 10^5$ | $4.38 \times 10^{-4}$ | 2 |
| VHH-COVE5 | $1.16 \times 10^{-8}$ | $9.11 \times 10^4$ | $1.06 \times 10^{-3}$ | 5 |
| VHH-COVE7 | $2.40 \times 10^{-8}$ | $7.33 \times 10^4$ | $1.86 \times 10^{-3}$ | 7 |
| VHH-COVE8 | $2.88 \times 10^{-8}$ | $5.53 \times 10^4$ | $1.59 \times 10^{-3}$ | 8 |
| VHH-COVE9 | $6.90 \times 10^{-9}$ | $3.86 \times 10^5$ | $2.67 \times 10^{-3}$ | 9 |

[Table 17]

| Binding affinity to RBD variant strain (E484K) | | | | |
|---|---|---|---|---|
| Antibody | $K_D$ (M) | $K_{on}$ (Ms$^{-1}$) | $K_{off}$ (s$^{-1}$) | SEQ ID NO: |
| VHH-COVE2 | $3.44 \times 10^{-8}$ | $1.06 \times 10^5$ | $3.65 \times 10^{-3}$ | 2 |
| VHH-COVE5 | $6.26 \times 10^{-7}$ | $3.92 \times 10^4$ | $2.46 \times 10^{-2}$ | 5 |
| VHH-COVE7 | $8.02 \times 10^{-8}$ | $8.81 \times 10^4$ | $7.07 \times 10^{-3}$ | 7 |
| VHH-COVE8 | $2.11 \times 10^{-7}$ | $6.97 \times 10^4$ | $1.47 \times 10^{-2}$ | 8 |
| VHH-COVE9 | $9.90 \times 10^{-9}$ | $3.10 \times 10^5$ | $3.07 \times 10^{-3}$ | 9 |

[Table 18]

| Binding affinity to RBD variant strain (N440K) | | | | |
|---|---|---|---|---|
| Antibody | $K_D$ (M) | $K_{on}$ (Ms$^{-1}$) | $K_{off}$ (s$^{-1}$) | SEQ ID NO: |
| VHH-COVE2 | $2.96 \times 10^{-9}$ | $1.66 \times 10^5$ | $4.91 \times 10^{-4}$ | 2 |
| VHH-COVE3 | $1.54 \times 10^{-8}$ | $2.92 \times 10^4$ | $4.48 \times 10^{-4}$ | 3 |
| VHH-COVE5 | $4.24 \times 10^{-8}$ | $4.21 \times 10^4$ | $1.78 \times 10^{-3}$ | 5 |
| VHH-COVE7 | $2.42 \times 10^{-8}$ | $4.66 \times 10^4$ | $1.13 \times 10^{-3}$ | 7 |
| VHH-COVE8 | $<1.0 \times 10^{-12}$ | $7.45 \times 10^4$ | $<1.0 \times 10^{-7}$ | 8 |
| VHH-COVE9 | $1.25 \times 10^{-8}$ | $2.06 \times 10^5$ | $2.58 \times 10^{-3}$ | 9 |

[Table 19]

| Binding affinity to BD variant strains (K417N, E484K, N501Y) | | | | |
|---|---|---|---|---|
| Antibody | $K_D$ (M) | $K_{on}$ (Ms$^{-1}$) | $K_{off}$ (s$^{-1}$) | SEQ ID NO: |
| VHH-COVE2 | $9.07 \times 10^{-8}$ | $5.84 \times 10^4$ | $5.29 \times 10^{-3}$ | 2 |
| VHH-COVE3 | $2.65 \times 10^{-7}$ | $4.00 \times 10^4$ | $1.06 \times 10^{-2}$ | 3 |
| VHH-COVE5 | $3.06 \times 10^{-7}$ | $2.04 \times 10^5$ | $6.25 \times 10^{-2}$ | 5 |
| VHH-COVE7 | $9.37 \times 10^{-8}$ | $4.24 \times 10^4$ | $3.98 \times 10^{-3}$ | 7 |
| VHH-COVE8 | $2.39 \times 10^{-7}$ | $7.99 \times 10^4$ | $1.91 \times 10^{-2}$ | 8 |
| VHH-COVE9 | $1.09 \times 10^{-8}$ | $1.46 \times 10^5$ | $1.59 \times 10^{-3}$ | 9 |

As the result of binding analysis, all VHH antibodies used in this test bound to each RBD variant strain. However, it was observed that VHH-COVE2, VHH-COVE3, VHH-COVE5, VHH-COVE7, and VHH-COVE8, which showed competivity of an epitope with ACE2, influence the binding activity depending on the position of the mutation. In contrast, in VHH-COVE9 that binds to an epitope different from ACE2, a reduction in the binding activity associated with these mutations was not recognized. This suggests a possibility that VHH-COVE9 shows neutralizing activity also to each variant strain above.

(Example 19) Measurement of binding activity of SARS-CoV-2 India variant strain to RBD

Regarding VHH-COVE3, which was confirmed to have binding activity to India variant strain-derived spike protein in Example 15, the binding activity to India variant strain-derived RBD was evaluated. First, Dynabeads (TM) MyOne Carboxylic Acid (Thermo Fisher Scientific) warmed to ordinary temperature for 30 minutes or more was sufficiently stirred, and 250 $\mu$L thereof was fractionated in a 1.5-mL tube. Subsequently, the supernatant was removed using a magnetic rack. After addition of 250 $\mu$L of 15 mM MES buffer (pH 6.0), vortex was performed for 10 seconds. Subsequently, the supernatant was removed using a magnetic rack. The beads were suspended in 50 $\mu$L of 15 mM MES buffer (pH 6.0) and mixed with 50 $\mu$L of 1-ethyl-3-(3-dimethylaminopropyl)carbodiimide hydrochloride adjusted to 10 mg/mL, followed by stirring at ordinary temperature for 30 minutes. Subsequently, the supernatant was removed using a magnetic rack. Subsequently, 120 $\mu$L of 15 mM MES buffer (pH 6.0) containing 50 ng of VHH-COVE3 was prepared, and the beads were suspended in the solution. After stirring at ordinary temperature overnight, the supernatant was removed using a magnetic rack. The beads were suspended in 500 $\mu$L of PBST and were stirred for 10 minutes. Subsequently, the supernatant was removed using a magnetic rack. This operation was repeated twice. The beads were suspended in 500 $\mu$L of PBST containing 3% bovine serum albumin, followed by stirring for 30 minutes. Subsequently, washing with PBST was performed three times. In accordance with the operation above, VHH-COVE3-immobilized beads were prepared. In addition, as a control, VHH-non-immobilized beads were also prepared. Subsequently, SARS-CoV-2 (2019-nCoV) spike RBD (L452R, E484Q) protein (His Tag) (hereinafter, referred to as "India type RBD") (Sino Biological, Inc.) was adjusted to 12.5 $\mu$g/mL with PBST. The VHH-COVE3-immobilized beads were suspended in 200 $\mu$L of India type RBD in a 1.5-mL tube, followed by slowly stirring. At 10 minutes after the start of stirring, 30 $\mu$L of the reaction solution was fractionated and was left to stand on a magnetic rack until the solution became transparent, and the supernatant was fractionated and used for SDS-PAGE. The SDS-PAGE was performed by applying a sample containing 20 $\mu$L of the supernatant to each well of Bolt (R) Bis-Tris Plus gel (Thermo Fisher Scientific) and then performing electrophoresis at 200 V for 35 minutes. As the molecular weight marker, Novex (TM) Sharp Pre-stained Protein Standard (Thermo Fisher Scientific) was used. In staining, GelCode (TM) Blue Safe Protein Stain (Thermo Fisher Scientific) was used.

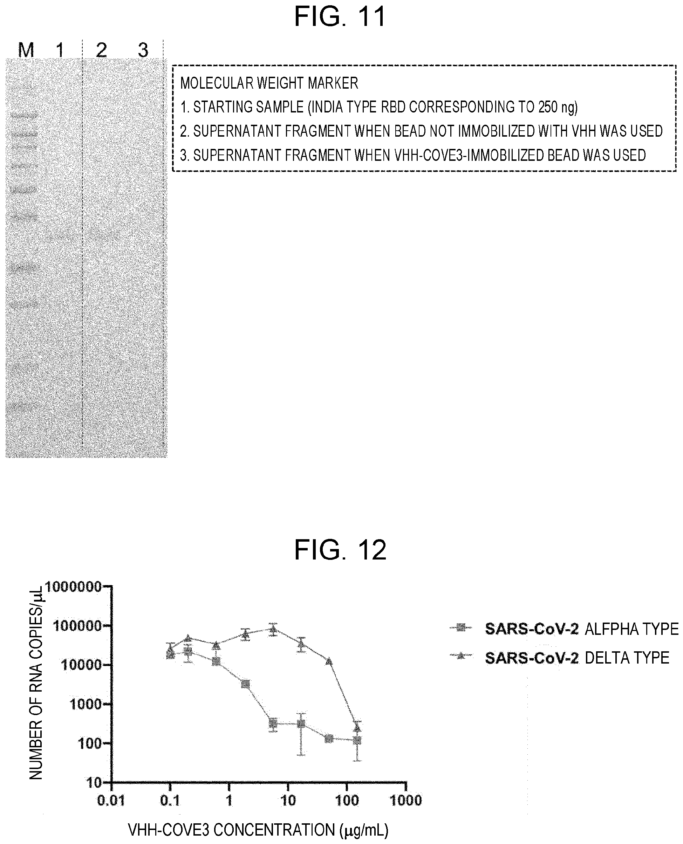

The results of SDS-PAGE are shown in Figure 11. As the starting sample, India type RBD corresponding to 250 ng was subjected to SDS-PAGE, and this band was used for comparison in the evaluation of the binding activity of the VHH. When VHH-non-immobilized beads were reacted with India type RBD as the control, a band was observed at the same position as that of the starting sample (comparison of lanes 1 and 2). That is, it was confirmed that there was no binding activity between the VHH-non-immobilized beads and India type RBD. In contrast, when VHH-COVE3-immobilized beads were reacted with India type RBD, the India type RBD-derived band disappeared (comparison of lanes 1 and 3). This result means that the VHH-COVE3-immobilized beads adsorbed India type RBD. Summarizing the above, it was revealed that VHH-COVE3 has clear binding activity to India type RBD.

(Example 20) Production of VHH-COVE9 variant by recombinant *Corynebacterium*

(1) Construction of VHH-COVE9 variant expression plasmid

Considering the sequences in the DNA library of R5 of in vitro selection, VHH-COVE9-R108K (SEQ ID NO: 106) which is provided by making, to VHH-COVE9 (SEQ ID NO: 73), a mutation of R108K in the CDR3 region and also VHH-COVE9-N109D (SEQ ID NO: 107) provided by making a mutation of N109D and VHH-COVE9-Y110S (SEQ ID NO: 108) provided by making a mutation of Y110S in the respective CDR3 regions were selected. In production of these VHHs by recombinant *Corynebacterium,* VHH-COVE9-R108K (SEQ ID NO: 109), VHH-COVE9-N109D (SEQ ID NO: 110), and VHH-COVE9-Y110S (SEQ ID NO: 111) were each expressed in a form of having an Ala residue attached to the N-terminal and a His tag sequence including a linker (SEQ ID NO: 74) attached to the C-terminal. Each VHH gene was synthesized by Eurofins Genomics K.K. and was introduced into an expression plasmid. The synthesized VHH clone genes and an empty expression plasmid were treated with two restriction enzymes, SfiI and ApaI (both available from Thermo Fisher Scientific), and DNAs of target fragments were each isolated and purified by agarose gel electrophoresis. Subsequently, the restriction enzyme-treated VHH clone gene and the expression plasmid were ligated using Ligation Mix (Toyobo Life Science), and the ligation product was transformed into cloning competent cell JM109 strain. The plasmid was extracted from the transformed *Escherichia coli* to obtain the expression plasmid of each VHH clone.

(2) Production of recombinant *Corynebacterium glutamicum* strain

The plasmid was introduced into *Corynebacterium glutamicum* strain by the same conditions as in Example 4.

(3) VHH production

The recombinant *Corynebacterium glutamicum* strain produced in (2) above was inoculated in CM2G culture medium containing 25 μg/mL kanamycin and was shaken at 30°C overnight to prepare a pre-culture solution. The pre-culture solution was inoculated at 5% in 700 μL of a PM1S culture medium placed in a 96-deep well plate and was shake-cultured at 25°C for 72 hours. After completion of the culture, centrifugation with a centrifugal separator (Thermo Fisher Scientific Scientific) was performed at 4,000 rpm at 4°C for 30 minutes to collect the culture supernatant in a tube.

The collected culture supernatant was filtered through PVDF membrane (Merck Millipore) and then purified using Ni-NTA agarose beads (FUJIFILM Wako Pure Chemical Corporation) in accordance with the manufacturer's instructions. The VHH was eluted by addition of 700 μL of His-tag elution buffer (50 mM Tris-HCl, 300 mM NaCl, 500 mM imidazole, pH 7.5) and centrifugation with a centrifugal separator (TOMY Company, Ltd.) at 500 ×g for 30 seconds. The eluate was collected as a VHH antibody sample.

In order to verify the purity of the purified VHH, SDS-PAGE was performed. 4 μL of a sample was applied to each well, and electrophoresis was then performed under conditions of 150 V for 1 hour. As the molecular weight marker, Precision Plus Protein (TM) Standards (BIO-RAD Laboratories, Inc.) were used. After the electrophoresis, Coomassie brilliant blue (CBB) staining of the gel was performed to verify that VHH was synthesized in the culture supernatant.

(Example 21) Measurement of binding activity by bio-layer interferometry

The binding activities of VHH-COVE9-R108K (SEQ ID NO: 106), VHH-COVE9-N109D (SEQ ID NO: 107), and VHH-COVE9-Y110S (SEQ ID NO: 108) to SARS-CoV-2 (2019-nCoV) spike SI-Fc recombinant protein (Sino Biological, Inc.) were measured by the same method as in Example 6. The equilibrium dissociation constant ($K_D$) of each VHH clone to spike S1 protein is shown in Table 20.

[Table 20]

| Antibody | $K_D$ (M) | $K_{on}$ (Ms$^{-1}$) | $K_{off}$ (s$^{-1}$) | SEQ ID NO: |
|---|---|---|---|---|
| VHH-COVE9-R108K | $3.28 \times 10^{-9}$ | $1.33 \times 10^5$ | $4.37 \times 10^{-4}$ | 106 |
| VHH-COVE9-N109D | $8.38 \times 10^{-9}$ | $1.26 \times 10^5$ | $1.06 \times 10^{-3}$ | 107 |
| VHH-COVE9-Y110S | $4.74 \times 10^{-9}$ | $1.44 \times 10^5$ | $6.83 \times 10^{-4}$ | 108 |

(Example 22) Measurement of neutralizing activity of VHH-COVE3 to SARS-CoV-2 variant strain

The neutralizing activities of *Bacillus subtilis*-derived VHH-COVE3 to SARS-CoV-2 alpha and delta variant strains were measured. The antibody was adjusted to a concentration of 150 μg/mL by mixing with 2% Fetal Bovine Serum (FBS)/Dulbecco Modified Eagle Medium (DMEM). Furthermore, 100 μL of VHH clone mixture solution and 100 μL of 2% FBS/DMEM were mixed to prepare 3-fold dilution series solution of the antibody.

100 μL of the 3-fold dilution series solution of the VHH clone was added to a 96-well plate, and 100 μL of a solution of $2.5 \times 10^6$ RNA copies/mL of SARS-CoV-2 alpha or delta variant strain (obtained from the National Institute of Infectious Diseases) was further added. Subsequently, incubation was performed at 37°C for 2 hours and then at 4°C overnight to contact the antibody with the virus. 100 μL of the solution in which the antibody and the virus were in contact was added to wells in which vero-E6/TMPRSS2 cells (purchased from JCRB Cell Bank) were inoculated, followed by culturing at 37°C in a 5% $CO_2$ environment. The culture supernatant was collected 1 day after the start of the culturing, and the number of viral genome copies in the supernatant was counted by quantitative PCR. The quantitative PCR used SARS-CoV-2 Detection Kit (Toyobo Life Science). 3 μL of the culture supernatant was mixed with 3 μL of a pretreatment solution, and 3 μL of the mixture was added to an RT-PCR reaction solution (reaction solution: 15 μL, enzyme solution: 2.5 μL, primer/probe solution: 2.5 μL), and quantitative PCR was performed with LightCycler (R) 96. The reaction conditions were reverse transcription reaction: 42°C, 5 minutes; pre-denaturation: 95°C, 10 seconds; and 45 cycles of cycle reaction [denaturation: 95°C, 1 second, association: 50°C, 3 seconds, extension: 55°C, 10 seconds] to perform detection.

The results of the number of viruses in the cells obtained by quantitative PCR are shown in Figure 12. The results revealed that VHH-COVE3 shows neutralizing activity to both SARS-CoV-2 alpha type and delta type and also shows binding activities to these viruses.

(Example 23) Measurement of competitive inhibition activity to binding of SARS-CoV-2 kappa variant strain-derived (L452R, E484Q, B.1.617.1 strain) and ACE2

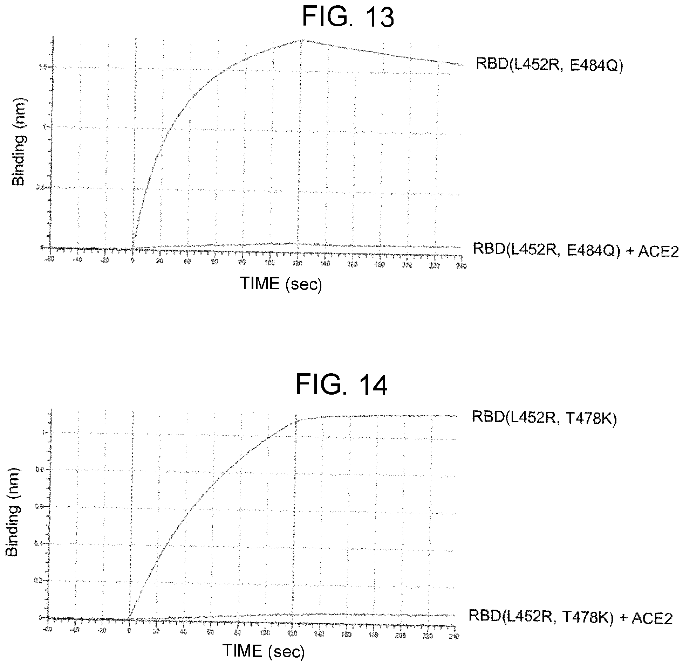

Competitive inhibition of *Bacillus subtilis*-derived VHH-COVE3 to the binding of SARS-CoV-2 kappa variant strain-derived RBD (L452R, E484Q) and human ACE2 was evaluated using Octet 384. VHH-COVE3 adjusted to 20 μg/mL with a kinetic buffer (PBS containing 0.05% Tween 20, pH 7.4) was immobilized on an Anti-Penta-HIS (HIS1K, ForteBio) sensor chip by incubation for 120 seconds and was immersed in each of 10 μg/mL SARS-CoV-2 spike RBD recombinant protein (SPD-C525e, AcroBiosystems) solution (50 μL), a mixture solution of 20 μg/mL SARS-CoV-2 spike RBD recombinant protein (25 μL) and 40 μg/mL ACE2 (SPD-C525e, AcroBiosystems) solution (25 μL), and 20 μg/mL ACE2 (50 μL, reference well) as analytes. After confirming the binding after 120 seconds, the sensor chip was immersed in the buffer solution for 120 seconds.
The results of data treatment using an ACE2-immobilized sensor chip as a reference are shown in Figure 13. As a result, the binding response between the VHH-COVE3 immobilized on the sensor chip and RBD clearly disappeared in the mixture solution of RBD and ACE2. Since VHH-COVE3 clearly inhibited the binding between ACE2 and kappa variant strain-derived RBD, it was demonstrated that VHH-COVE3 has neutralizing activity also to the kappa variant strain.

(Example 24) Measurement of competitive inhibition activity to binding of SARS-CoV-2 delta variant strain-derived RBD (L452R, T478K, B.1.617.2 strain) and ACE2

Competitive inhibition of *Bacillus subtilis*-derived VHH-COVE3 to the binding of SARS-CoV-2 delta variant strain-derived RBD (L452R and T478K mutant strain) and human ACE2 was evaluated using Octet 384. VHH-COVE3 adjusted to 20 μg/mL with a kinetic buffer (PBS containing 0.05% Tween 20, pH 7.4) was immobilized on an Anti-Penta-HIS (HIS1K, ForteBio) sensor chip by incubation for 120 seconds and was immersed in each of 10 μg/mL SARS-CoV-2 spike RBD recombinant protein (SPD-C525e, AcroBiosystems) solution (50 μL), a mixture solution of 20 μg/mL SARS-CoV-2 spike RBD recombinant protein (25 μL) and 40 μg/mL ACE2 (SPD-C525e, AcroBiosystems) solution (25 μL), and 20 μg/mL ACE2 (50 μL) as analytes. After confirming the binding after 120 seconds, the sensor chip was immersed in the buffer solution for 120 seconds.
The results of data treatment using an ACE2-immobilized sensor chip as a reference are shown in Figure 14. As a result, the binding response between the VHH-COVE3 immobilized on the sensor chip and RBD clearly disappeared in the mixture solution of RBD and ACE2. Since VHH-COVE3 clearly inhibited the binding between ACE2 and delta variant strain-derived RBD, it was demonstrated that VHH-COVE3 has neutralizing activity also to the delta variant strain.

(Example 25) Measurement of neutralizing activity of VHH-COVE9 to SARS-CoV-2 alpha variant strain in hamster body

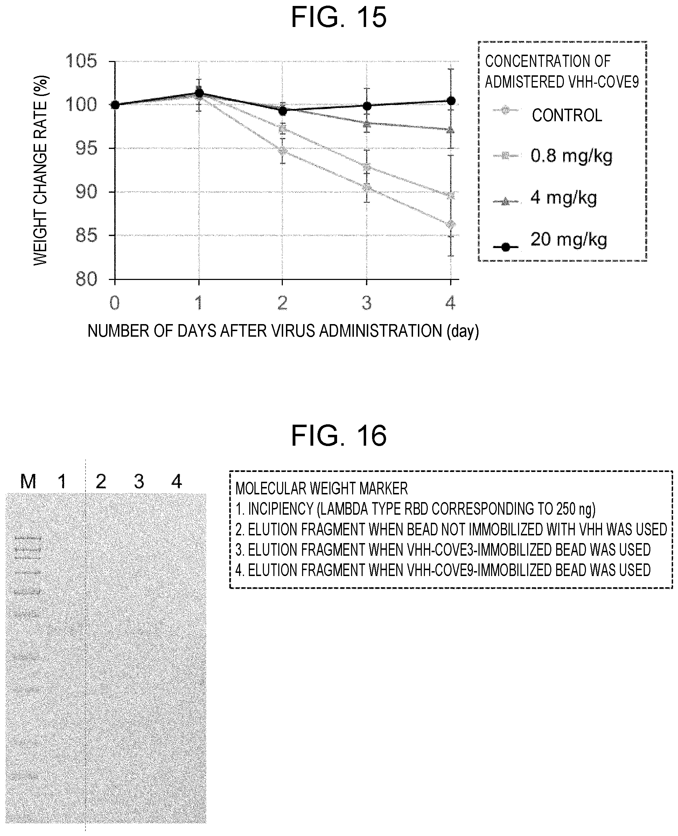

Triple anesthetic (mixture of medetomidine hydrochloride: 0.15 mg/kg, midazolam: 2.0 mg/kg, and butorphanol tartrate: 2.5 mg/kg) was intraperitoneally administered to twelve 6-week old male Syrian hamsters (hereinafter, hamsters) for sedation, and SARS-CoV-2 alpha variant strain ($10^{3.5}$ TCID$_{50}$/head) was then transnasally administered. After the transnasal administration, atipamezole was intraperitoneally administered to the hamsters to recover from the sedation. One day after the administration of SARS-CoV-2, the triple anesthetic was intraperitoneally administered to all hamsters again for sedation, and VHH-COVE9 was then transnasally administered to three hamsters for each of different concentrations (20 mg/kg, 4 mg/kg, and 0.8 mg/kg). As a control, only the PBS containing albumin used for dilution of VHH-COVE9 was transnasally administered to the remaining three hamsters. The weight of each hamster was measured daily from immediately after the virus administration to 4 days after the administration.
The results of evaluation of neutralizing activity of VHH-COVE9 in hamster bodies are shown in Figure 15. In the control in which VHH-COVE9 was not administered, a clear weight loss associated with SARS-CoV-2 infection was observed. In administration of 0.8 mg/kg of VHH-COVE9, alleviation of weight loss was observed. When 4 mg/kg or 20 mg/kg of VHH-COVE9 was administered, the weight loss associated with SARS-CoV-2 infection was obviously suppressed. That is, it was revealed that VHH-COVE9 has neutralizing activity (therapeutic effect) to SARS-CoV-2 alpha variant strain.

(Example 26) Measurement of binding activity of SARS-CoV-2 lambda variant strain to RBD

The binding activity of VHH-COVE3 to lambda variant strain-derived RBD was evaluated. VHH-COVE3-immobilized beads were prepared in accordance with Example 19. As controls, VHH-non-immobilized beads and VHH-COVE9-immobilized beads were also prepared. Subsequently, SARS-CoV-2 (2019-nCoV) spike RBD (L452Q, F490S) protein (His Tag) (hereinafter, referred to as "lambda type RBD") (Sino Biological, Inc.) was adjusted to 12.5 μg/mL using PBST.

VHH-COVE3-immobilized beads, VHH-COVE9-immobilized beads, and VHH-non-immobilized beads were each suspended in 200 µL of lambda type RBD in a 1.5-mL tube and were slowly stirred. At 10 minutes after the start of stirring, 100 µL of the reaction solution was fractionated and was left to stand on a magnetic rack until the solution became transparent, and the supernatant was then removed. Subsequently, the collected beads were suspended in 40 µL of distilled water and then mixed with the sample buffer of SDS-PAGE. The mixture was left to stand at 100°C for 5 minutes to elute the lambda type RBD adsorbed to the beads in the sample buffer to prepare elution fractions. SDS-PAGE was performed by applying 40 µL of each sample containing the elution fraction to each well of Bolt (R) Bis-Tris Plus gel (Thermo Fisher Scientific) and then performing electrophoresis at 200 V for 35 minutes. As the molecular weight marker, Novex (TM) Sharp Pre-stained Protein Standard (Thermo Fisher Scientific) was used. In staining, GelCode (TM) Blue Safe Protein Stain (Thermo Fisher Scientific) was used.

The results of SDS-PAGE are shown in Figure 16. As the starting sample, lambda type RBD corresponding to 250 ng was subjected to SDS-PAGE (lane 1). In the elution fractions of VHH-non-immobilized beads as a control and VHH-COVE9-immobilized beads, no band derived from lambda type RBD was observed (lanes 2 and 4). In contrast, in the elution fraction of VHH-COVE3-immobilized beads, a band derived from lambda type RBD was observed (lane 3). The results above indicate that VHH-COVE3-immobilized beads adsorbed lambda type RBD. That is, it was revealed that VHH-COVE3 has binding activity to lambda type RBD.

(Example 27) Measurement of neutralizing activity of VHH-COVE3 to SARS-CoV-2 alpha type mutant strain in hamster body

Triple anesthetic (mixture of medetomidine hydrochloride: 0.15 mg/kg, midazolam: 2.0 mg/kg, and butorphanol tartrate: 2.5 mg/kg) was intraperitoneally administered to six 6-week old male Syrian hamsters (hereinafter, hamsters) for sedation, and SARS-CoV-2 alpha variant strain ($10^{3.5}$ TCID$_{50}$/head) was then transnasally administered. After the transnasal administration, atipamezole was intraperitoneally administered to the hamsters to recover from the sedation. One day after the administration of SARS-CoV-2, the triple anesthetic was intraperitoneally administered to all hamsters again for sedation, and 8 mg/kg of VHH-COVE3 was then transnasally administered to three hamsters. As a control, only the PBS containing albumin used for dilution of VHH-COVE3 was transnasally administered to the remaining three hamsters. The weight of each hamster was measured daily from immediately after the virus administration to 4 days after the administration.

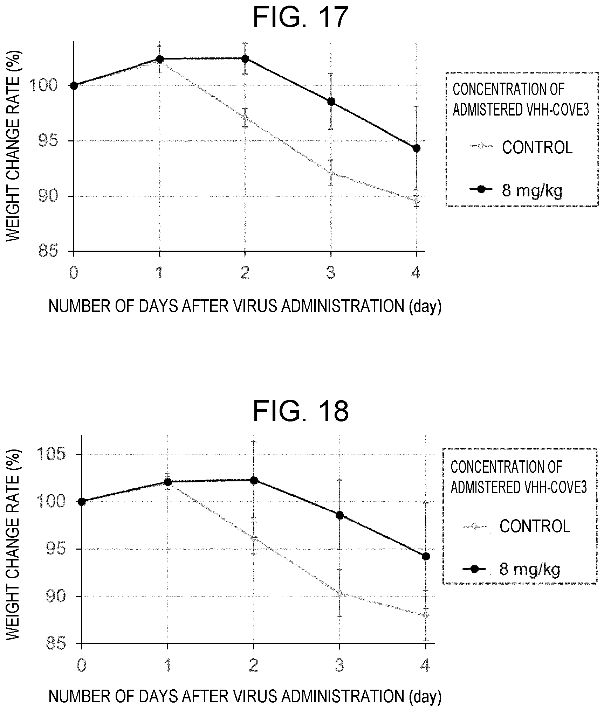

The results of evaluation of neutralizing activity of VHH-COVE3 in the hamster bodies are shown in Figure 17. In the control in which VHH-COVE3 was not administered, a clear weight loss associated with SARS-CoV-2 infection was observed. The weight loss was suppressed by administration of 8 mg/kg of VHH-COVE3. That is, it was revealed that VHH-COVE3 has neutralizing activity (therapeutic effect) to SARS-CoV-2 alpha variant strain.

(Example 28) Measurement of neutralizing activity of VHH-COVE3 to SARS-CoV-2 delta variant strain in hamster body

Triple anesthetic (mixture of medetomidine hydrochloride: 0.15 mg/kg, midazolam: 2.0 mg/kg, and butorphanol tartrate: 2.5 mg/kg) was intraperitoneally administered to six 6-week old male Syrian hamsters (hereinafter, hamsters) for sedation, and SARS-CoV-2 delta variant strain ($10^{3.5}$ TCID$_{50}$/head) was then transnasally administered. After the transnasal administration, atipamezole was intraperitoneally administered to the hamsters to recover from the sedation. One day after the administration of SARS-CoV-2, the triple anesthetic was intraperitoneally administered to all hamsters again for sedation, and 8 mg/kg of VHH-COVE3 was then transnasally administered to three hamsters. As a control, only the PBS containing albumin used for dilution of VHH-COVE3 was transnasally administered to the remaining three hamsters. The weight of each hamster was measured daily from immediately after the virus administration to 4 days after the administration.

The results of evaluation of neutralizing activity of VHH-COVE3 in the hamster bodies are shown in Figure 18. In the control in which VHH-COVE3 was not administered, a clear weight loss associated with SARS-CoV-2 infection was observed. The weight loss was suppressed by administration of 8 mg/kg of VHH-COVE3. That is, it was revealed that VHH-COVE3 has neutralizing activity (therapeutic effect) to SARS-CoV-2 delta variant strain.

Industrial Applicability

The present invention is useful in the fields of medicament and diagnostic agent.

**Claims**

1. A SARS-CoV-2-binding peptide comprising one or more structural domains comprising CDR3 consisting of an amino acid sequence of any of SEQ ID NOs: 1 to 9 or an amino acid sequence obtained by substituting at least one amino acid in the amino acid sequence with another amino acid.

2. The peptide according to claim 1, wherein the SARS-CoV-2-binding peptide is one selected from the group consisting of a VHH antibody, a heavy-chain antibody, and a VHH antibody multimer.

3. The peptide according to claim 2, wherein the VHH antibody multimer is a multimer having a plurality of the structural domains linked to each other.

4. The peptide according to any of claims 1 to 3, wherein the amino acid sequence of any of SEQ ID NOs: 1 to 9 has a KD of $3.6 \times 10^{-9}$ M or less for SARS-CoV-2.

5. The peptide according to any of claims 1 to 4, wherein the structural domain further comprises: CDR1 consisting of an amino acid sequence of any of SEQ ID NOs: 10 to 18 or an amino acid sequence obtained by substituting at least one amino acid in the amino acid sequence with another amino acid; and CDR2 consisting of an amino acid sequence of any of SEQ ID NOs: 19 to 27 or an amino acid sequence obtained by substituting at least one amino acid in the amino acid sequence with another amino acid.

6. The peptide according to claim 5, selected from 1) to 9), the peptide comprising one or more structural domains comprising each of CDR1, CDR2, and CDR3 consisting of an amino acid sequence of SEQ ID NOs: 1 to 27 shown below or an amino acid sequence obtained by substituting at least one amino acid in the amino acid sequence with another amino acid.

|    | CDR1          | CDR2          | CDR3          |
|----|---------------|---------------|---------------|
| 1) | SEQ ID NO: 10 | SEQ ID NO: 19 | SEQ ID NO: 1  |
| 2) | SEQ ID NO: 11 | SEQ ID NO: 20 | SEQ ID NO: 2  |
| 3) | SEQ ID NO: 12 | SEQ ID NO: 21 | SEQ ID NO: 3  |
| 4) | SEQ ID NO: 13 | SEQ ID NO: 22 | SEQ ID NO: 4  |
| 5) | SEQ ID NO: 14 | SEQ ID NO: 23 | SEQ ID NO: 5  |
| 6) | SEQ ID NO: 15 | SEQ ID NO: 24 | SEQ ID NO: 6  |
| 7) | SEQ ID NO: 16 | SEQ ID NO: 25 | SEQ ID NO: 7  |
| 8) | SEQ ID NO: 17 | SEQ ID NO: 26 | SEQ ID NO: 8  |
| 9) | SEQ ID NO: 18 | SEQ ID NO: 27 | SEQ ID NO: 9  |

7. A nucleic acid encoding the peptide according to claim 1.

8. A method for detecting SARS-CoV-2 in a sample, comprising a step of contacting the peptide according to any one of claims 1 to 6 with a test sample.

9. A kit for detecting SARS-CoV-2 comprising the peptide according to any one of claims 1 to 6.

10. A medicament comprising the peptide according to any one of claims 1 to 6.

11. The medicament according to claim 10 for preventing or treating SARS-CoV-2 infectious disease.

12. Use of the peptide according to any one of claims 1 to 6 for manufacturing a medicament.

13. The use according to claim 12, wherein the medicament is a medicament for preventing or treating SARS-CoV-2 infectious disease.

14. The peptide according to any one of claims 1 to 6 for use as a medicament.

15. The peptide according to claim 14, wherein the medicament is a medicament for preventing or treating SARS-CoV-2 infectious disease.

16. A method for preventing or treating SARS-CoV-2 infectious disease comprising administering the peptide according to any one of claims 1 to 6 to a subject in need thereof.

# FIG. 1

# FIG. 2

FIG. 3

# FIG. 4

VHH-COVE1 + S1 PROTEIN
S1 PROTEIN

VHH-COVE3 + S1 PROTEIN
VHH-COVE2 + S1 PROTEIN

VHH-COVE9

# FIG. 5

IC50=0.05μg/mL

FIG. 6

## FIG. 7

## FIG. 8

# FIG. 9

# FIG. 10

# FIG. 11

MOLECULAR WEIGHT MARKER
1. STARTING SAMPLE (INDIA TYPE RBD CORRESPONDING TO 250 ng)
2. SUPERNATANT FRAGMENT WHEN BEAD NOT IMMOBILIZED WITH VHH WAS USED
3. SUPERNATANT FRAGMENT WHEN VHH-COVE3-IMMOBILIZED BEAD WAS USED

# FIG. 12

SARS-CoV-2 ALFPHA TYPE

SARS-CoV-2 DELTA TYPE

## FIG. 13

RBD(L452R, E484Q)

RBD(L452R, E484Q) + ACE2

## FIG. 14

RBD(L452R, T478K)

RBD(L452R, T478K) + ACE2

# FIG. 15

CONCENTRATION OF ADMISTERED VHH-COVE9

- CONTROL
- 0.8 mg/kg
- 4 mg/kg
- 20 mg/kg

(y-axis) WEIGHT CHANGE RATE (%)

(x-axis) NUMBER OF DAYS AFTER VIRUS ADMINISTRATION (day)

# FIG. 16

MOLECULAR WEIGHT MARKER
1. INCIPIENCY (LAMBDA TYPE RBD CORRESPONDING TO 250 ng)
2. ELUTION FRAGMENT WHEN BEAD NOT IMMOBILIZED WITH VHH WAS USED
3. ELUTION FRAGMENT WHEN VHH-COVE3-IMMOBILIZED BEAD WAS USED
4. ELUTION FRAGMENT WHEN VHH-COVE9-IMMOBILIZED BEAD WAS USED

# FIG. 17

NUMBER OF DAYS AFTER VIRUS ADMINISTRATION (day)

# FIG. 18

NUMBER OF DAYS AFTER VIRUS ADMINISTRATION (day)

## INTERNATIONAL SEARCH REPORT

| International application No. |
| --- |
| **PCT/JP2021/036446** |

**A.    CLASSIFICATION OF SUBJECT MATTER**

*C12N 15/13*(2006.01)i; *A61K 39/215*(2006.01)i; *A61P 31/14*(2006.01)i; *C07K 16/10*(2006.01)i; *C07K 16/46*(2006.01)i
FI:   C12N15/13 ZNA; A61K39/215; A61P31/14; C07K16/10 ZNA; C07K16/46

According to International Patent Classification (IPC) or to both national classification and IPC

**B.    FIELDS SEARCHED**

Minimum documentation searched (classification system followed by classification symbols)

C12N15/13; A61K39/215; A61P31/14; C07K16/10; C07K16/46

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C.    DOCUMENTS CONSIDERED TO BE RELEVANT**

| Category* | Citation of document, with indication, where appropriate, of the relevant passages | Relevant to claim No. |
| --- | --- | --- |
| X | CN 111303279 A (INSTITUTE OF PATHOGEN BIOLOGY CAMS) 19 June 2020 (2020-06-19)<br>    examples 1, 2, table 1, paragraph [0063] | 1-16 |
| X | WU, Yanling et al. Fully human single-domain antibodies against SARS-CoV-2. bioRxiv, 31 March 2020, doi:https://doi.org/10.1101/2020.03.30.015990<br>    abstract, fig. 2, 3, S1b, S4, lines 68-77 | 1-16 |
| P, X | BERTOGLIO, Federico et al. A SARS-CoV-2 neutralizing antibody selected from COVID-19 patients binds to the ACE2-RBD interface and is tolerant to most known RBD mutations. Cell Reports, 27 July 2021, vol. 36, No. 109433<br>    abstract, page 5, line 7 to page 6, line 3, fig. 4 | 1-16 |
| P, X | GUETTLER, Thomas et al. Neutralization of SARS-CoV-2 by highly potent, hyperthermostable, and mutation-tolerant nanobodies. The EMBO Journal, 2021.8.9, vol. 40, e107985<br>    abstract, page 13, right row, fourth paragraph to page 15, right row, first paragraph, fig. 9 | 1-16 |

☐ Further documents are listed in the continuation of Box C.          ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **22 November 2021** | **07 December 2021** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**EP 4 223 878 A1**

**INTERNATIONAL SEARCH REPORT**

| International application No. |
| --- |
| **PCT/JP2021/036446** |

| Box No. I | Nucleotide and/or amino acid sequence(s) (Continuation of item 1.c of the first sheet) |
| --- | --- |

1.  With regard to any nucleotide and/or amino acid sequence disclosed in the international application, the international search was carried out on the basis of a sequence listing:

    a.  ☑  forming part of the international application as filed:

          ☑  in the form of an Annex C/ST.25 text file.

          ☐  on paper or in the form of an image file.

    b.  ☐  furnished together with the international application under PCT Rule 13*ter*.1(a) for the purposes of international search only in the form of an Annex C/ST.25 text file.

    c.  ☐  furnished subsequent to the international filing date for the purposes of international search only:

          ☐  in the form of an Annex C/ST.25 text file (Rule 13*ter*.1(a)).

          ☐  on paper or in the form of an image file (Rule 13*ter*.1(b) and Administrative Instructions, Section 713).

2.  ☐  In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

## INTERNATIONAL SEARCH REPORT
### Information on patent family members

International application No.

**PCT/JP2021/036446**

| Patent document cited in search report | Publication date (day/month/year) | Patent family member(s) | Publication date (day/month/year) |
|---|---|---|---|
| CN 111303279 A | 19 June 2020 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014158430 A
- JP 2006174707 A
- JP 4336082 B

**Non-patent literature cited in the description**

- Neutralizing nanobodies bind SARS-CoV-2 spike RBD and block interaction with ACE2. *Nature Structural & Molecular Biology,* 2020
- Development of multi-specific humanized llama antibodies blocking SARS-CoV-2/ACE2 interaction with high affinity and avidity. *Emerging Microbes & Infections,* 2020, vol. 9, 1034-1036
- Structural Basis for Potent Neutralization of Betacoronaviruses by Single-Domain Camelid Antibodies. *Cell,* 2020, vol. 181, 1004-1015
- Identification of Human Single-Domain Antibodies against SARS-CoV-2. *Cell Host & Microbe,* 2020, vol. 27, 891-898
- SARS-CoV-2 spike S1 protein. Sino Biological, Inc,
- SARS-CoV-2 spike S1 protein. Sino Biological, Inc